# EUROPÄISCHE PATENTSCHRIFT

(11) **EP 1 831 326 B1**
(45) Veröffentlichungstag und Bekanntmachung des Hinweises auf die Patenterteilung: **01.03.2017**
(21) Anmeldenummer: 05850295.6
(22) Anmeldetag: 16.12.2005
(51) Int. Cl.: C09K 11/06, C08G 61/10, C07C 15/00, H01L 51/30

(54) **ELEKTROLUMINESZIERENDE POLYMERE UND DEREN VERWENDUNG**
ELECTROLUMINESCENT POLYMERS AND THEIR USE
POLYMERES ELECTROLUMINESCENTS ET LEUR UTILISATION

(30) Priorität: 18.12.2004 EP 04030093
(43) Veröffentlichungstag der Anmeldung: 12.09.2007
(73) Patentinhaber: Merck Patent GmbH, 64293 Darmstadt (DE)
(72) Erfinder: BÜSING, Arne, 65929 Frankfurt (DE); STÖSSEL, Phillip, 60487 Frankfurt am Main (DE)
(86) Internationale Anmeldenummer: PCT/EP2005/013610
(87) Internationale Veröffentlichungsnummer: WO 2006/063852

(56) Entgegenhaltungen:
- JP-A- 2004 014 187
- JP-A- 2004 335 415
- ZHENG L ET AL: "A BINAPHTHYL-BASED CONJUGATED POLYMER FOR LIGHT-EMITTING DIODES" CHEMISTRY OF MATERIALS, AMERICAN CHEMICAL SOCIETY, WASHINGTON, US, Bd. 12, Nr. 1, Januar 2000 (2000-01), Seiten 13-15, XP000919559 ISSN: 0897-4756 in der Anmeldung erwähnt
- JEN ALEX K-Y ET AL: "Efficient light-emitting diodes based on a binaphthalene-containing polymer" APPLIED PHYSICS LETTERS, AMERICAN INSTITUTE OF PHYSICS. NEW YORK, US, Bd. 75, Nr. 24, 13. Dezember 1999 (1999-12-13), Seiten 3745-3747, XP012024232 ISSN: 0003-6951
- HU Q-S ET AL: "CONJUGATED POLYMERS WITH MAIN CHAIN CHIRALITY. 2. SYNTHESIS OF OPTICALLY ACTIVE POLYARYLENES" MACROMOLECULES, AMERICAN CHEMICAL SOCIETY. EASTON, US, Bd. 29, Nr. 15, 15. Juli 1996 (1996-07-15), Seiten 5075-5082, XP000596740 ISSN: 0024-9297 in der Anmeldung erwähnt
- ZHAN XIAOWEI ET AL: "New series of blue-emitting and electron-transporting copolymers based on cyanostilbene" CHEM. MATER.; CHEMISTRY OF MATERIALS MAY 20 2003, Bd. 15, Nr. 10, 20. Mai 2003 (2003-05-20), Seiten 1963-1969, XP001206289
- DATABASE CA [Online] CHEMICAL ABSTRACTS SERVICE, COLUMBUS, OHIO, US; LECUYER, A. PAGE ET AL: "Graphite inserted with bromine. Solid bromination reagent in organic chemistry" XP002328076 gefunden im STN Database accession no. 1973:515342 & BULLETIN DE LA SOCIETE CHIMIQUE DE FRANCE , (5) (PT. 2), 1690-2 CODEN: BSCFAS; ISSN: 0037-8968, 1973,
- DATABASE CA [Online] CHEMICAL ABSTRACTS SERVICE, COLUMBUS, OHIO, US; KIM, JI IN ET AL: "Enantioselective catalysis of the triplex Diels-Alder reaction: a study of scope and mechanism" XP002328077 gefunden im STN Database accession no. 1993:6465 & JOURNAL OF THE AMERICAN CHEMICAL SOCIETY , 114(24), 9309-17 CODEN: JACSAT; ISSN: 0002-7863, 1992,

## Beschreibung

Seit einigen Jahren läuft eine breit angelegte Forschung zur Kommerzialisierung von Anzeige- und Beleuchtungselementen auf Basis polymerer (organischer) Leuchtdioden (PLEDs). Ausgelöst wurde diese Entwicklung durch die Grundlagenentwicklungen, welche in WO 90/13148 offenbart sind. Seit kurzem sind auch erste Produkte (kleine Anzeigen in einem Rasierapparat und einem Mobiltelefon der Fa. PHILIPS N.V.) im Markt erhältlich. Allerdings sind immer noch deutliche Verbesserungen der verwendeten Materialien nötig, um diese Displays zu einer echten Konkurrenz zu den derzeit marktbeherrschenden Flüssigkristallanzeigen (LCD) zu machen.

Als Polymere für vollfarbige Anzeigeelemente (Full-Colour-Displays) wurden verschiedene Materialklassen entwickelt. So kommen beispielsweise Poly-Fluoren-, Poly-Spirobifluoren-, Poly-Dihydrophenanthren-, Poly-Indenofluoren- und Poly-Phenanthren-Derivate in Betracht oder auch Polymere, die eine Kombination dieser Strukturelemente enthalten. Im Allgemeinen sind für derartigen Einsatz Polymere, welche Poly-para-phenylen (PPP) als Strukturelement enthalten, möglich.

Die Polymere gemäß dem Stand der Technik zeigen zum Teil schon gute Eigenschaften. Trotz der bereits erzielten Fortschritte entsprechen sie allerdings noch nicht den Anforderungen, die an sie für hochwertige PLED-Anwendungen gestellt werden. Insbesondere ist die Effizienz der emittierenden Polymere für viele Anwendungen noch nicht ausreichend. Auch die Kombination von Polymeren mit Triplett-Emittern führt bislang noch nicht zu den erhofften hohen Effizienzen, insbesondere bei grüner Emission. Ein weiteres Problem der Polymere gemäß dem Stand der Technik ist deren häufig geringe Löslichkeit. So können mit den oben genannten Polymerklassen nur dann lösliche Polymere erhalten werden, wenn diese durch Alkyl- und/oder Alkoxyketten solubilisiert werden. Manche dieser Einheiten, insbesondere Dihydrophenanthren- und Phenanthren-Einheiten weisen trotz dieser Substitutionen nur eine geringe Löslichkeit in den üblicherweise verwendeten Lösemitteln auf. Daher können diese entweder nur aus ökologisch bedenklichen und nicht in technischen Prozessierungsschritten verwendbaren Lösemitteln wie Chlorbenzol verarbeitet werden, oder es finden sich überhaupt keine geeigneten Lösemittel, so dass hier Abhilfe nötig ist. Auch die Substitution durch lange Alkyl- und/oder Alkoxyketten ist nicht wünschenswert, da diese keine elektronische Funktion wahrnehmen, jedoch möglicherweise den Ladungstransport zwischen den Polymerketten hemmen und die Konzentration der funktionellen Einheiten im Polymer reduzieren. Weiterhin wirken lange Alkyl- und Alkoxyketten als Weichmacher, die die Glastemperatur der Polymere senken, was für die Anwendung in organischen elektronischen Vorrichtungen nicht erwünscht ist.

Ohne an eine bestimmte Theorie gebunden sein zu wollen, vermuten wir, dass die elektronischen Eigenschaften der durchgängig konjugierten Polymere gemäß dem Stand der Technik noch nicht optimal für einen ausbalancierten Ladungstransport sind. Dadurch ist möglicherweise der Elektronen- bzw. der Lochstrom in der Vorrichtung zu groß, und es kann sich kein ausbalanciertes Ladungsgleichgewicht einstellen. Außerdem kann es für manche Anwendungen sinnvoll sein, die Funktionen der unterschiedlichen Einheiten im Polymer, also beispielsweise Ladungstransport und Emission, vollständig oder teilweise voneinander zu trennen. Dies ist in vollständig konjugierten Polymeren nur dadurch möglich, dass beispielsweise die Ladungstransporteinheiten in die Seitenkette des Polymers eingebaut werden, wie in EP 1263834 beschrieben. Diese Funktionen in der Seitenkette weisen jedoch keine konjugierten Abschnitte außer der funktionellen Einheit selber auf, was wiederum für einen guten Ladungstransport nicht immer ausreichend ist.

Es wurde nun überraschend gefunden, dass eine neue Klasse von Polymeren, bei der die Konjugation durch den Einsatz bestimmter, neuer Monomereinheiten reduziert, aber nicht vollkommen unterbrochen ist, sehr gute und den oben genannten Stand der Technik übertreffende Eigenschaften aufweist. Diese Polymere und deren Verwendung in PLEDs sind daher Gegenstand der vorliegenden Erfindung. Die neuen Struktureinheiten eignen sich insbesondere als Polymer-Grundgerüst, besonders in Verbindung mit einem anderen Polymer-Grundgerüst, welches durch die Verwendung der neuartigen Einheiten eine deutlich bessere Löslichkeit aufweist, oder aber auch in Verbindung mit Triplett-Emittern.

Die Verwendung bestimmter Binaphthyl-Einheiten in Polymeren ist in der Literatur beschrieben:
So beschreiben X. Wu et al. (Synth. Metals 2001, 121, 1699-1700) ein alternierendes Copolymer aus 6,6'-verknüpften 2,2'-substituierten 1,1'-Binaphthyleinheiten und substituierten Phenyleneinheiten. Die gute Löslichkeit des Polymers wird dabei auf die Anwesenheit von vier n-Hexyloxy-Seitenketten zurückgeführt, was darauf hindeutet, dass entsprechende unsubstituierte Einheiten nicht zu löslichen Polymeren führen. Elektrolumineszenzergebnisse werden nicht vorgestellt. L. Zheng et al. (Chem. Mater. 2000, 12, 13-15) beschreiben ein Poly(binaphthylvinylen-phenylenvinylen), in dem die 1,1'-Binaphthyleinheiten wiederum über die 6,6'-Position im Polymer verknüpft sind. Auch hier sind wieder Alkoxyketten für die Löslichkeit des Polymers notwendig. Das Polymer zeigt in Lösung hellblaue Fluoreszenz, im Film jedoch bathochrom verschobene grünblaue Emission, so dass es nicht für blaue Emission geeignet ist. Außerdem ist die externe Quanteneffizienz mit 0.1 % sehr niedrig und die Einsatzspannung mit 6 V sehr hoch. Weitere ähnliche Polymere sind in der Literatur bekannt (z. B. K. Y. Musick et al., Macromolecules 1998, 31, 2933; Q.-S. Hu et al., Macromolecules 1996, 29, 1082; Q.-S. Hu et al., Macromolecules 1996, 29, 5075; L. Ma et al., Macromolecules 1996, 29, 5083; A. K.-Y. Jen, Appl. Phys. Lett. 1999, 75, 3745). 6,6'-verknüpfte 1,1'-Binaphthyleinheiten scheinen allgemein den Schwachpunkt aufzuweisen, dass die Löslichkeit dieser Polymere unzureichend ist und gute Löslichkeit nur durch die Einführung langer Alkyl- oder Alkoxyketten erreicht wird. Diese wiederum tragen, wie oben bereits erwähnt, nichts zur elektronischen Funktion des Polymers bei und können sich sogar störend auf den Ladungstransport zwischen den Polymerketten und die Glasübergangstemperatur auswirken.
U. Anton und K. Müllen (Macromolecules 1993, 26, 1248) beschreiben Polynaphthaline, wobei als Monomere unsubstituiertes 4,4'-Binaphthyl und alkyl-substituiertes 1,5-Dibromnaphthalin eingesetzt wird. Dabei ist selbst bei Anwesenheit von Hexylketten als Substituenten am Dibromnaphthalin die Löslichkeit immer noch so gering, dass nur Oligomere, jedoch keine Polymere erhalten werden. Selbst mit zwei Dodecylketten werden nur niedrige Molekulargewichte (Mₙ = 5900 g/mol) erhalten. Die Verwendung derartiger Oligomere in organischen elektronischen Vorrichtungen ist nicht beschrieben.
In DE 4024647 werden 4,4'-verknüpfte Oligo- und Poly-naphthaline beschrieben, die sich als Materialien zur Wärmeisolation und als Elektrodenmaterialien eignen. Eine Bevorzugung für eine Substitution in 2,2'-Position ist nicht zu erkennen. Die Verwendung dieser Polymere in organischen Leuchtdioden bzw. als Halbleitermaterial in anderen organischen elektronischen Vorrichtungen ist nicht beschrieben.
P. V. Bedworth und J. M. Tour (Macromolecules 1994, 27, 622-624) beschreiben helikale Oligomere, basierend auf enantiomerenreinen substituierten 4,4'-verknüpften 2,2'-Dimethoxy-1,1'-binaphthylen (M_{w} = 14700 g/mol). Eine Eignung für organische elektronische Vorrichtungen lässt sich nicht erkennen. Durch die Dimethoxy-Substitution ist eine derartige Eignung auch nicht zu erwarten, da die Methoxygruppen einerseits vermutlich die thermische Stabilität der Polymere herabsetzen und andererseits die Emissionsfarbe bathochrom verschieben, so dass diese Polymere für viele Anwendungen nicht geeignet sein werden. Für die synthetische Zugänglichkeit dieser Polymere scheint die Anwesenheit der Methoxygruppen jedoch unumgänglich zu sein.
V. Percec et al. (Polymer Bulletin 1992, 29, 271-276) beschreiben ein alternierendes Polymer aus unsubstituiertem 4,4'-(1,1'-Binaphthyl) und 4,4'-(3,3'-Diphenyl)biphenyl. Dabei werden allerdings nur Polymerisationsgrade von weniger als 10 erreicht, so dass die erhaltenen Strukturen eher als Oligomer bezeichnet werden sollten. Die Anwendung in organischen elektronischen Vorrichtungen ist nicht beschrieben, aber es ist nicht zu vermuten, dass sich Oligomere mit so geringem Molekulargewicht dafür eignen.
X. Zhan et al., (Chem. Mater. 2003, 15, 1963-1969) beschreiben blau emittierende Polymere, basierend auf Cyanostilbenen, die durch 3,3'-(1,1'-Binaphthyl) verknüpft sind. Dabei wird Löslichkeit durch Einführung von Hexoxy-Ketten in der 2,2'-Position des Binaphthyls erreicht. Diese Polymere zeigen in organischen elektronischen Vorrichtungen jedoch nur schlechte Ergebnisse. So werden für die besten Vorrichtungen nur geringe Helligkeiten bei einer Spannung von 18 V erzielt und eine Quanteneffizienz von nur 0.2 %. Daher eignen sich diese Polymere nicht für die technische Anwendung.

Die Substitution der Binaphthyl-Einheiten in 2- bzw. 2,2'-Position mit Alkyl- bzw. Arylgruppen und die Verknüpfung im Polymer in der 4,4'-Position hat sich überraschend als besonders geeignet im Vergleich zur Verwendung unsubstituierter bzw. anders substituierter Binaphthyl-Einheiten und zur Verknüpfung über andere Positionen erwiesen. Dadurch lassen sich besonders gut lösliche Polymere mit besseren optischen und elektronischen Eigenschaften synthetisieren. Daher sind Polymere, die derartige Einheiten enthalten, Gegenstand der vorliegenden Erfindung.

Zur Übersicht sind im folgenden Schema die Positionen des 1,1'-Binaphthyls aufgezeigt: 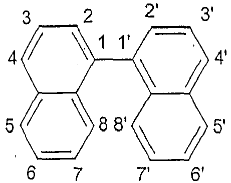 Gegenstand der Erfindung sind Polymere, enthaltend mindestens 1 mol%, bevorzugt mindestens 5 mol%, besonders bevorzugt mindestens 10 mol% einer ersten Wiederholeinheit gemäß Formel (1), 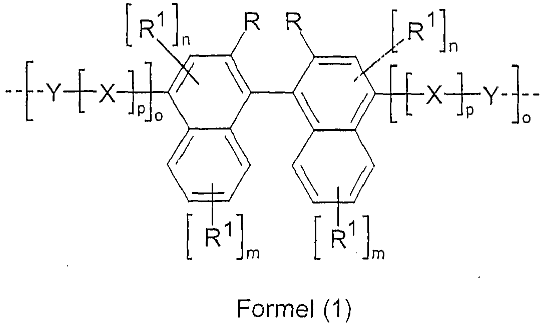 wobei die verwendeten Symbole und Indizes folgende Bedeutung besitzen:
- R: ist bei jedem Auftreten gleich oder verschieden H, eine geradkettige Alkylkette mit 1 bis 40 C-Atomen oder eine verzweigte oder cyclische Alkylkette mit 3 bis 40 C-Atomen, die jeweils durch R¹ substituiert sein kann und in der auch ein oder mehrere nicht benachbarte C-Atome durch N-R¹, O, S, O-CO-O, CO-O, -CR¹=CR¹- oder -C≡C- ersetzt sein können, mit der Maßgabe, dass die Heteroatome nicht direkt an die Naphthyl-Einheit gebunden sind, und in der auch ein oder mehrere H-Atome durch F, Cl, Br, I oder CN ersetzt sein können, oder ein aromatisches oder heteroaromatisches Ringsystem mit 5 bis 40 aromatischen Ringatomen, welches auch durch einen oder mehrere Reste R¹ substituiert sein kann; dabei können die beiden Reste R miteinander auch ein weiteres Ringsystem bilden; mit der Maßgabe, dass mindestens einer der beiden Reste R ungleich H ist;
- X: ist bei jedem Auftreten gleich oder verschieden -CR¹=CR¹-, -C≡C- oder N-Ar;
- Y: ist bei jedem Auftreten gleich oder verschieden ein bivalentes aromatisches oder heteroaromatisches Ringsystem mit 5 bis 40 aromatischen Ringatomen, welches durch einen oder mehrere Reste R¹ substituiert oder unsubstituiert sein kann;
- R¹: ist bei jedem Auftreten gleich oder verschieden H, F, Cl, Br, I, CN, N(R²)₂, Si(R²)₃, B(R²)₂, eine geradkettige Alkyl- oder Alkoxykette mit 1 bis 40 C-Atomen öder eine verzweigte oder cyclische Alkyl- oder Alkoxykette mit 3 bis 40 C-Atomen, in der jeweils auch ein oder mehrere nicht benachbarte C-Atome durch N-R², O, S, O-CO-O, CO-O, -CR¹=CR¹- oder -C≡C- ersetzt sein können und in der auch ein oder mehrere H-Atome durch F, Cl, Br, I oder CN ersetzt sein können, oder eine Aryl-, Aryloxy- oder Heteroarylgruppe mit 5 bis 40 aromatischen Ringatomen, welche auch durch einen oder mehrere nicht-aromatische Reste R¹ substituiert sein kann; dabei können auch zwei oder mehrere der Reste R¹ miteinander ein Ringsystem bilden;
- R²: ist bei jedem Auftreten gleich oder verschieden H oder ein aliphatischer oder aromatischer Kohlenwasserstoffrest mit 1 bis 20 C-Atomen;
- Ar: ist bei jedem Auftreten gleich oder verschieden ein monovalentes aromatisches oder heteroaromatisches Ringsystem mit 5 bis 40 aromatischen Ringatomen, welches mit R¹ substituiert oder unsubstituiert sein kann;
- n: ist bei jedem Auftreten gleich oder verschieden 0 oder 1;
- m: ist bei jedem Auftreten gleich oder verschieden 0, 1, 2, 3 oder 4;
- o: ist bei jedem Auftreten gleich oder verschieden 0, 1 oder 2;
- p: ist bei jedem Auftreten gleich oder verschieden 0 oder 1;
die gestrichelte Bindung bedeutet dabei in Formel (1) ebenso wie in allen weiteren Formeln die Verknüpfung im Polymer; und außerdem enthaltend mindestens 1 mol% einer zweiten Wiederholeinheit, die entweder gleich einer Wiederholeinheit gemäß Formel (1) ist oder verschieden ist.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer C₁- bis C₄₀-Alkoxygruppe werden besonders bevorzugt Methoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen System mit 1-30 C-Atomen, welches noch jeweils mit den oben genannten Resten R¹ substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Pyren, Chrysen, Perylen, Fluoranthen, Tetracen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einem aromatischen bzw. heteroaromatischen Ringsystem im Sinne dieser Erfindung soll ein System verstanden werden, das nicht notwendigerweise nur aromatische bzw. heteroaromatische Gruppen enthält, sondern in dem auch mehrere aromatische bzw. heteroaromatische Gruppen durch eine kurze nicht-aromatische Einheit (< 10 % der von H verschiedenen Atome, bevorzugt < 5 % der von H verschiedenen Atome), wie beispielsweise sp³-hybridisierter C, N, etc., unterbrochen sein können. So sollen also beispielsweise auch Systeme wie 9,9'-Spirobifluoren, 9,9-Diarylfluoren, Triarylamin, etc. als aromatische Ringsysteme verstanden werden. Dabei enthalten die aromatischen Gruppen mindestens 6 C-Atome und die heteroaromatischen Gruppen mindestens 2 C-Atome und mindestens ein Heteroatom, wobei die Summe aus C-Atomen und Heteroatomen mindestens 5 ergibt.

Einheiten gemäß Formel (1) liegen in zwei enantiomeren Formen vor. Dabei ist es gleichermaßen erfindungsgemäß, das Racemat, also die 1 : 1 Mischung der beiden Enantiomeren, oder eines der beiden Enantiomeren in angereicherter oder isolierter Form zu verwenden. Die Abfolge der Stereozentren in Polymeren wird als Taktizität bezeichnet.

In einem Aspekt der Erfindung handelt es sich um konjugierte Polymere. In einem weiteren Aspekt der Erfindung handelt es sich um teilkonjugierte Polymere. In nochmals einem weiteren Aspekt der Erfindung handelt es sich um nicht-konjugierte Polymere. Bevorzugt sind konjugierte oder teilkonjugierte Polymere.

Konjugierte Polymere im Sinne dieser Erfindung sind Polymere, die in der Hauptkette hauptsächlich sp²-hybridisierte Kohlenstoffatome, die auch durch entsprechende Heteroatome ersetzt sein können, enthalten. Dies bedeutet im einfachsten Fall abwechselndes Vorliegen von Doppel- (oder auch Dreifach-) und Einfachbindungen in der Hauptkette. Hauptsächlich meint, dass natürlich auftretende Defekte, die zu Konjugationsunterbrechungen führen, den Begriff "konjugiertes Polymer" nicht entwerten. Des Weiteren wird in diesem Anmeldetext ebenfalls als konjugiert bezeichnet, wenn sich in der Hauptkette beispielsweise Arylamineinheiten und/oder konjugierte Heterocyclen (d. h. Konjugation über N-, O-oder S-Atome) und/oder metallorganische Komplexe (d. h. Konjugation über das Metallatom) befinden. Hingegen würden Einheiten wie beispielsweise einfache Alkylbrücken, (Thio)Ether-, Ester-, Amid- oder Imidverknüpfungen eindeutig als nicht-konjugierte Segmente definiert. Unter einem teilkonjugierten Polymer soll ein Polymer verstanden werden, in dem längere konjugierte Abschnitte in der Hauptkette durch nicht-konjugierte Abschnitte unterbrochen sind, bzw. das längere konjugierte Abschnitte in den Seitenketten eines in der Hauptkette nicht-konjugierten Polymers enthält.
Es sei allerdings an dieser Stelle darauf verwiesen, dass Einheiten gemäß Formel (1) die Konjugation einer ansonsten durchgängig konjugierten Polymerkette verringern, da die beiden Naphthyleinheiten einen Winkel in der Größenordnung von 60 bis 120° miteinander bilden. Die Konjugation wird durch derartige Einheiten jedoch nicht vollständig unterbrochen.

Bevorzugt sind erfindungsgemäße Polymere, bei denen für Einheiten gemäß Formel (1) gilt:
- R: ist bei jedem Auftreten gleich oder verschieden H, eine geradkettige Alkylkette mit 1 bis 10 C-Atomen oder eine verzweigte oder cyclische Alkylkette mit 3 bis 10 C-Atomen, in der jeweils auch ein oder mehrere nicht benachbarte C-Atome durch -CH=CH- oder -C≡C- ersetzt sein können und in der auch ein oder mehrere H-Atome durch F oder CN ersetzt sein können, oder ein aromatisches oder heteroaromatisches Ringsystem mit 5 bis 20 aromatischen Ringatomen, weiches auch durch einen oder mehrere nicht-aromatische Reste R¹ substituiert sein kann; dabei können die beiden Reste R zusammen auch ein weiteres Ringsystem bilden; mit der Maßgabe, dass mindestens ein Rest R ungleich H ist;
- Y: ist bei jedem Auftreten gleich oder verschieden eine bivalente Aryl- oder Heteroarylgruppe mit 5 bis 25 aromatischen Ringatomen, welche durch einen oder mehrere Reste R¹ substituiert sein kann;
- Ar: ist bei jedem Auftreten gleich oder verschieden eine monovalente Aryl- oder Heteroarylgruppe mit 5 bis 20 aromatischen Ringatomen, welche mit R¹ substituiert oder unsubstituiert sein kann;
- R¹: ist bei jedem Auftreten gleich oder verschieden H, F, N(R²)₂, eine geradkettige Alkylkette mit 1 bis 10 C-Atomen oder eine verzweigte Alkylkette mit 3 bis 10 C-Atomen, in der jeweils auch ein oder mehrere nicht benachbarte C-Atome durch -CR¹=CR¹- oder -C≡C- ersetzt sein können und in der auch ein oder mehrere H-Atome durch F ersetzt sein können, oder eine Aryl- oder Heteroarylgruppe mit 5 bis 20 aromatischen Ringatomen, welche auch durch einen oder mehrere nicht-aromatische Reste R¹ substituiert sein kann; dabei können auch zwei oder mehrere der Reste R¹ miteinander ein Ringsystem bilden;
- n: ist bei jedem Auftreten gleich 0;
- m: ist bei jedem Auftreten gleich oder verschieden 0, 1 oder 2;
- o: ist bei jedem Auftreten gleich oder verschieden 0 oder 1;
die weiteren Symbole und Indizes sind wie oben unter Formel (1) definiert.

Besonders bevorzugt sind erfindungsgemäße Polymere, bei denen für Einheiten gemäß Formel (1) gilt:
- R: ist bei jedem Auftreten gleich oder verschieden H oder eine aromatische oder heteroaromatische Gruppe mit 5 bis 15 aromatischen Ringatomen, welche auch durch einen oder mehrere nicht-aromatische Reste R¹ substituiert sein kann; dabei können die beiden Reste R zusammen auch ein weiteres Ringsystem bilden; mit der Maßgabe, dass mindestens einer der Reste R ungleich H ist;
- X: ist bei jedem Auftreten gleich oder verschieden -CH=CH-, -C≡C- oder N-Ar;
- Y: ist bei jedem Auftreten gleich oder verschieden eine bivalente Aryl- oder Heteroarylgruppe mit 6 bis 15 C-Atomen, welche durch einen oder mehrere nicht-aromatische Reste R¹ substituiert sein kann;
- Ar: ist bei jedem Auftreten gleich oder verschieden eine monovalente Aryl- oder Heteroarylgruppe mit 5 bis 15 aromatischen Ringatomen, welche mit nicht-aromatischen Resten R¹ substituiert sein kann;
- n: ist bei jedem Auftreten gleich 0;
- m: ist bei jedem Auftreten gleich 0;
- o: ist bei jedem Auftreten gleich oder verschieden 0 oder 1;
die weiteren Symbole und Indizes sind wie oben definiert.

Die Bevorzugung aromatischer Reste R lässt sich durch die besseren elektronischen Eigenschaften und die höhere thermische Stabilität der Polymere begründen.

In einer bevorzugten Ausführungsform der Erfindung sind die beiden Reste R gleich. In einer weiteren bevorzugten Ausführungsform der Erfindung ist einer der beiden Reste R gleich H und der andere ein aromatisches oder heteroaromatisches Ringsystem, wie oben beschrieben.

Ebenso ist es möglich, dass die Substituenten X und Y jeweils gleich sind, bzw. auch unterschiedlich sein sind oder nur einseitig auftreten.

Die erfindungsgemäßen Polymere enthalten neben Einheiten gemäß Formel (1) bevorzugt noch weitere Strukturelemente, die verschieden von Einheiten gemäß Formel (1) sind. Bevorzugt sind die weiteren Strukturelemente konjugiert. Hier sei vor allem auch auf die relativ umfangreichen Auflistungen in WO 02/077060, in WO 05/014689 und die darin aufgeführten Zitate verwiesen. Die weiteren Struktureinheiten sind bevorzugt aus den im Folgenden beschriebenen Klassen ausgewählt:
Gruppe 1: Aromatische Einheiten, welche üblicherweise das Polymer-Grundgerüst darstellen:
   Das Polymer-Grundgerüst dient im Allgemeinen als "Matrix" für die funktionellen Einheiten im Polymer, wie beispielsweise Ladungstransport- oder Emissionseinheiten. Einheiten dieser Gruppe sind aromatische, carbocyclische Strukturen mit 6 bis 40 C-Atomen, die substituiert oder unsubstituiert sein können, wobei als Substituenten die oben genannten Reste R¹ in Frage kommen. Hier kommen Fluoren-Derivate (z. B. EP 0842208, WO 99/54385, WO 00/22027, WO 00/22026, WO 00/46321) in Betracht. Des Weiteren sind auch Spirobifluoren-Derivate (z. B. EP 0707020, EP 0894107, WO 03/020790) eine Möglichkeit. Auch Polymere, die eine Kombination dieser beiden Monomer-Einheiten enthalten, wurden bereits vorgeschlagen (WO 02/077060). In WO 05/014689 sind Dihydrophenanthren-Derivate beschrieben. Weiterhin kommen cis- oder trans-Indenofluoren-Derivate (z. B. WO 04/041901, WO 04/113412) in Frage, aber auch 1,4-Phenylen-Derivate, 4,4'-Biphenylylen-Derivate, 4,4"-Terphenylylen-Derivate, 2,7-Phenanthren-Derivate (z. B. DE 0102004020298.2), Dihydropyren- oder Tetrahydropyren-Derivate und weitere nicht explizit aufgeführte aromatische Strukturen. Einheiten aus Gruppe 1 sind also bevorzugt ausgewählt aus der Gruppe der Fluoren-Derivate, der Spirobifluoren-Derivate, der Dihydrophenanthren-Derivate, der cis- oder trans-Indenofluoren-Derivate, der 1,4-Phenylen-Derivate, der 4,4'-Biphenylylen-Derivate, der 4,4"-Terphenylylen-Derivate, der 2,7-Phenanthren-Derivate, der Dihydropyren- oder Tetrahydropyren-Derivate. Besonders bevorzugte Einheiten aus dieser Gruppe sind ausgewählt aus Spirobifluoren, Fluoren, Dihydrophenanthren, cis-Indenofluoren, trans-Indenofluoren und 2,7-Phenanthren, die durch R¹ substituiert oder unsubstituiert sein können.
Gruppe 2: Einheiten, welche die Morphologie bzw. die Emissionsfarbe verändern:
   Bevorzugt sind diese Einheiten ausgewählt aus der Gruppe der durch R¹ substituierten oder unsubstituierten kondensierten aromatischen Strukturen mit 6 bis 40 C-Atomen oder Tolan-, Stilben- oder Bisstyrylarylenderivaten, insbesondere 1,4-Naphthylen-, 1,4- oder 9,10-Anthrylen-, 1,6- oder 2,7- oder 4,9-Pyrenylen-, 3,9-oder 3,10-Perylenylen-, 4,4'-Tolanylen-, 4,4'-Stilbenylen- oder 4,4"-Bisstyrylarylenderivate.
Gruppe 3: Einheiten, welche die Lochinjektions- und/oder -transporteigenschaften der Polymere erhöhen:
   Dies sind im Allgemeinen aromatische Amine oder Phosphine oder elektronenreiche Heterocyclen. Diese sind bevorzugt ausgewählt aus der Gruppe der durch R¹ substituierten oder unsubstituierten Triarylamine, Benzidine, N,N,N',N'-Tetraaryl-para-phenylendiamine, Triarylphosphine, Phenothiazine, Phenoxazine, Dihydrophenazine, Thianthrene, Dibenzo-p-dioxine, Phenoxathiine, Carbazole, Azulene, Thiophene, Pyrrole, Furane und weiteren O-, S- oder N-haltigen Heterocyclen mit hoch liegendem HOMO (HOMO = höchstes besetztes Molekülorbital). Diese Einheiten können in die Hauptkette oder in die Seitenkette des Polymers eingebaut werden. Je nach Struktur ist auch das Polymergrundgerüst in der Lage, ausreichend gut Löcher zu leiten, so dass nicht notwendigerweise Einheiten aus Gruppe 3 anwesend sein müssen.
Gruppe 4: Einheiten, welche die Elektroneninjektions- und/oder -transporteigenschaften der Polymere erhöhen:
   Dies sind im Allgemeinen elektronenarme Aromaten oder Heterocyclen. Diese sind bevorzugt ausgewählt aus der Gruppe der durch R¹ substituierten oder unsubstituierten Pyridine, Pyrimidine, Pyridazine, Pyrazine, Triazine, Oxadiazole, Chinoline, Chinoxaline oder Phenazine, aber auch Verbindungen wie Triarylborane und weitere O-, S- oder N-haltige Heterocyclen mit niedrig liegendem LUMO (LUMO = niedrigstes unbesetztes Molekülorbital) kommen in Frage. Je nach Struktur ist auch das Polymergrundgerüst selbst in der Lage, ausreichend gut Elektronen zu leiten, so dass nicht notwendigerweise Einheiten aus Gruppe 4 vorhanden sein müssen.
Gruppe 5: Einheiten, die Kombinationen von Einzeleinheiten der Gruppe 3 und Gruppe 4 aufweisen:
   Es kann bevorzugt sein, wenn in den erfindungsgemäßen Polymeren Einheiten enthalten sind, in denen Strukturen, welche Lochtransporteigenschaften, und Strukturen, welche Elektronentransporteigenschaften aufweisen, direkt aneinander gebunden sind, also Strukturen aus den oben genannten Gruppen 3 und 4. Viele dieser Einheiten verschieben die Emissionsfarbe ins Grüne, Gelbe oder Rote; ihre Verwendung eignet sich also für die Erzeugung anderer Emissionsfarben aus ursprünglich blau emittierenden Polymeren.
Gruppe 6: Einheiten, die aus dem Triplett-Zustand Licht emittieren: Struktureinheiten aus dieser Gruppe können auch bei Raumtemperatur mit hoher Effizienz aus dem Triplettzustand Licht emittieren und zeigen Elektrophosphoreszenz statt Elektrofluoreszenz. Hierfür eignen sich zunächst Verbindungen, welche Schweratome mit einer Ordnungszahl von mehr als 36 enthalten. Besonders geeignet sind Verbindungen, welche d- oder f-Übergangsmetalle enthalten, die diese Bedingung erfüllen. Ganz besonders bevorzugt sind Struktureinheiten, welche Elemente der Gruppe 8 bis 10 (Ru, Os, Rh, Ir, Pd, Pt) enthalten, insbesondere Ir oder Pt. Diese Metallkomplexe können in die Hauptkette und/oder in die Seitenkette des Polymers gebunden werden. Als geeignet hat sich auch der Einbau derartiger Metallkomplexe an Verzweigungspunkte im Polymer erwiesen, wie beispielsweise in DE 102004032527.8 beschrieben. Wenn Einheiten aus Gruppe 6 vorhanden sind, kann es bevorzugt sein, gleichzeitig auch Einheiten aus Gruppe 7 zu verwenden. Jedoch auch ohne derartige Einheiten aus Gruppe 7 können mit Triplett-Emittern sehr hohe Effizienzen erreicht werden.
Gruppe 7: Einheiten, welche den Transfer vom Singulett- zum Triplett-Zustand unterstützen:
   Für den Einsatz von Triplett-Emittern kann es bevorzugt sein, unterstützend weitere Strukturelemente einzusetzen, welche den Übergang vom Singulett- zum Triplett-Zustand und damit die Elektrophosphoreszenzeigenschaften verbessern. Hierfür kommen beispielsweise Carbazoleinheiten in Frage, wie in WO 04/070772 und WO 04/113468 beschrieben, aber auch Keto-, Phosphinoxid-, Sulfoxid- oder Sulfon-Einheiten, wie in der nicht offen gelegten Anmeldung DE 10349033.7 beschrieben, oder Silaneinheiten, wie in WO 05/040302 beschrieben. Die Einheiten aus Gruppe 7 sind also bevorzugt ausgewählt aus der Gruppe der Carbazole, der überbrückten Carbazol-Dimere, der Ketone, Phosphinoxide, Sulfoxide, Sulfone oder der Silane.

Bevorzugt sind Polymere, die neben Struktureinheiten gemäß Formel (1) zusätzlich noch eine oder mehrere Einheiten ausgewählt aus den Gruppen 1 bis 7 enthalten. Dabei kann es auch vorteilhaft sein, wenn gleichzeitig mehr als eine Struktureinheit aus einer der Gruppen 1 bis 7 vorliegt.

Besonders bevorzugt sind Polymere, die neben Einheiten gemäß Formel (1) noch Einheiten aus der Gruppe 1 enthalten, ganz besonders bevorzugt mindestens 30 mol% dieser Einheiten.
Bevorzugt sind weiterhin Polymere, die neben Einheiten gemäß Formel (1) noch Einheiten aus der Gruppe 3 und/oder 4 enthalten, besonders bevorzugt aus Gruppe 3, ganz besonders bevorzugt mindestens 5 mol% dieser Einheiten.

Bevorzugt beträgt der Anteil Einheiten gemäß Formel (1) mindestens 5 mol%, besonders bevorzugt mindestens 10 mol%, ganz besonders bevorzugt mindestens 20 mol%. Diese Bevorzugung gilt vor allem, wenn es sich bei den Einheiten gemäß Formel (1) um das Polymer-Grundgerüst handelt. Bei anderen Funktionen können andere Anteile bevorzugt sein. Für andere Anwendungen, beispielsweise für organische Transistoren, kann der bevorzugte Anteil nochmals unterschiedlich sein, beispielsweise bis zu 100 mol%, wenn es sich um lochleitende Einheiten gemäß Formel (1) handelt.

Die erfindungsgemäßen Polymere weisen in der Regel 10 bis 10000, bevorzugt 50 bis 5000, besonders bevorzugt 50 bis 2000 Wiederholeinheiten auf.

Die Löslichkeit der Polymere wird teilweise durch die Substituenten R¹ an den weiteren anwesenden Struktureinheiten gewährleistet. Wie jedoch oben bereits beschrieben, sind lange Alkyl- bzw. Alkoxy-Substituenten im Polymer nicht immer wünschenswert und führen auch nicht immer zur gewünschten Löslichkeit. Auch die Substituenten R und, falls vorhanden, R¹ an Formel (1) tragen zur Löslichkeit der Polymere bei. Jedoch trägt bereits die Grundstruktur der Formel (1) selbst zu einer erheblich besseren Löslichkeit des Polymers bei, so dass das Vorhandensein von langkettigen Substituenten R¹ an den Naphthyleinheiten nicht nötig und auch nicht bevorzugt ist. Das Vorhandensein von einer oder zwei Gruppen R ist für eine gute Löslichkeit ausreichend und erhöht auch deutlich die Löslichkeit des gesamten Polymers. Dabei ist es nicht nötig, dass R lange Alkylketten enthält, sondern es ist ausreichend, wenn hier entweder rein aromatische Substituenten verwendet werden oder aromatische Substituenten, die mit nur kurzen Alkylketten, beispielsweise tert-Butyl, substituiert sind oder kurzkettige Alkylsubstituenten. Auch Polymere, in denen nur einer der Reste R eine Gruppe ungleich Wasserstoff darstellt, weisen eine sehr gute Löslichkeit auf.

Je nach Substitutionsmuster eignen sich Einheiten gemäß Formel (1) für verschiedene Funktionen im Polymer. So können diese Einheiten bevorzugt als Polymer-Grundgerüst bzw. in Kombination mit einem Polymer-Grundgerüst, wie oben beschrieben, zur Steigerung der Löslichkeit oder auch als Lochleiter oder als Emitter eingesetzt werden. Das Polymer-Grundgerüst dient im Allgemeinen als "Matrix" für die funktionellen Einheiten im Polymer, wie beispielsweise Ladungstransport- oder Emissionseinheiten. Welche Verbindungen sich insbesondere für welche Funktion eignen, ist vor allem durch die Gruppen X und Y beschrieben. Die Substituenten R und R¹ haben einen weniger ausgeprägten Einfluss auf die Funktion der Einheiten gemäß Formel (1).

So gilt für die Verwendung als Polymer-Grundgerüst bzw. in Kombination mit einem Polymergrundgerüst zur Steigerung der Löslichkeit bevorzugt:
p ist bei jedem Auftreten gleich 0,
d. h. es handelt sich um eine rein aromatische Struktureinheit.

Für die Verwendung von Einheiten gemäß Formel (1) als lochtransportierende Einheiten gilt bevorzugt:
p ist bei jedem Auftreten gleich oder verschieden 0 oder 1, wobei mindestens ein p = 1 ist;
o ist bei jedem Aufteten gleich oder verschieden 0, 1 oder 2, wobei o ungleich 0 ist, wenn das entsprechende p = 1 ist;
X ist bei jedem Auftreten N-Ar;
d. h. es handelt sich um Triarylaminderivate des Binaphthyls.

Für die Verwendung von Einheiten gemäß Formel (1) als Emitter gilt bevorzugt:
p ist bei jedem Auftreten gleich oder verschieden 0 oder 1, wobei mindestens ein p = 1 ist;
o ist bei jedem Auftreten gleich oder verschieden 0, 1 oder 2, wobei o ungleich 0 ist, wenn das entsprechende p = 1 ist;
X ist bei jedem Auftreten gleich oder verschieden -CR¹=CR¹-, -C≡C- oder N-Ar, wobei mindestens ein X gleich -CR¹=CR¹- oder -C≡C- ist,
d. h. es handelt sich um Diarylvinylen- oder Diarylacetylenderivate des Binaphthyls im weitesten Sinne, die auch noch zusätzlich Triarylamineinheiten enthalten können.

Beispiele für bevorzugte Einheiten gemäß Formel (1) sind Strukturen gemäß den abgebildeten Beispielen (1) bis (21),wobei die Verknüpfung im Polymer jeweils über die 4,4'-Positionen der Binaphthyleinheiten erfolgt, wie über die gestrichelten Bindungen angedeutet. Mögliche weitere Substituenten R¹ sind für die bessere Übersichtlichkeit im Allgemeinen nicht bzw. nicht überall aufgeführt. Dabei sind die Beispiele (1) bis (9) Beispiele für Grundgerüst-Einheiten, die Beispiele (10) bis (17) Beispiele für emittierende Einheiten und die Beispiele (18) bis (21) Beispiele für lochleitende Einheiten.

| | | |
|---|---|---|
| 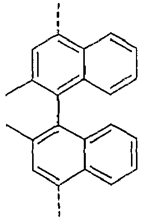 | 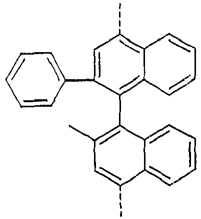 | 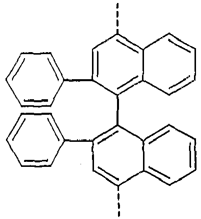 |
| Beispiel 1 | Beispiel 2 | Beispiel 3 |
| 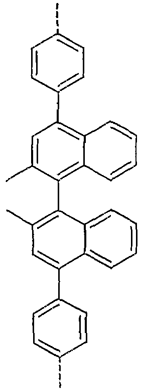 | 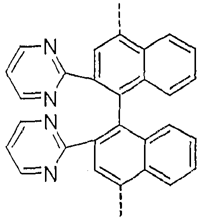 | 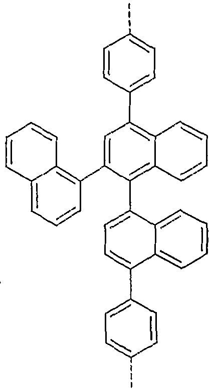 |
| Beispiel 4 | Beispiel 5 | Beispiel 6 |
| 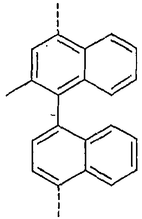 | 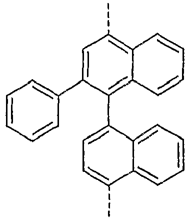 | 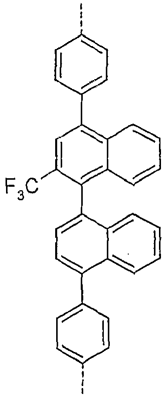 |
| Beispiel 7 | Beispiel 8 | Beispiel 9 |
| 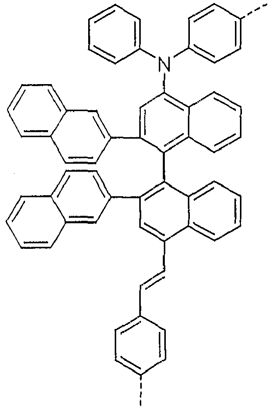 | 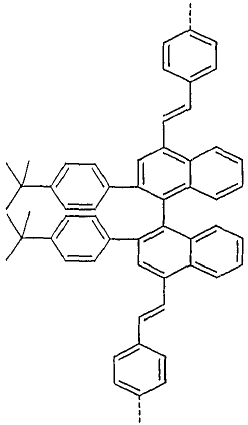 | 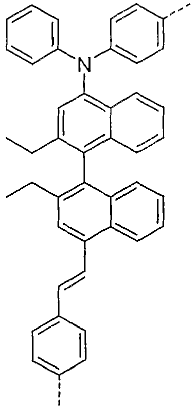 |
| Beispiel 10 | Beispiel 11 | Beispiel 12 |
| 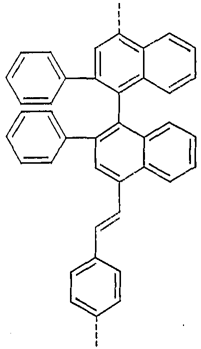 | 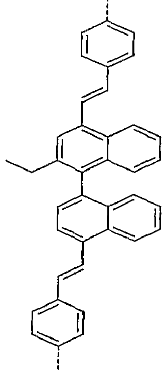 | 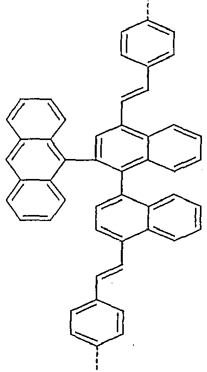 |
| Beispiel 13 | Beispiel 14 | Beispiel 15 |
| 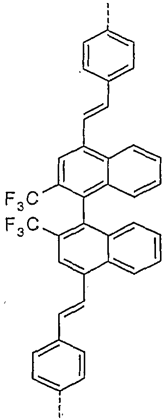 | 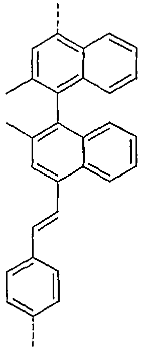 | 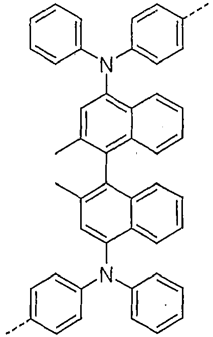 |
| Beispiel 16 | Beispiel 17 | Beispiel 18 |
| 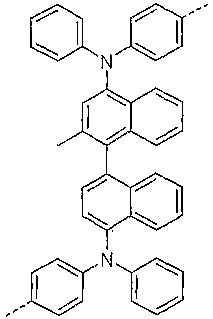 | 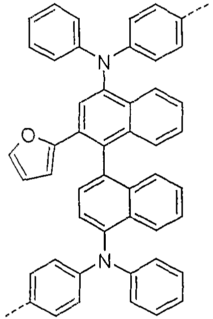 | 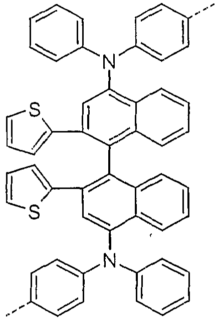 |
| Beispiel 19 | Beispiel 20 | Beispiel 21 |

Die erfindungsgemäßen Polymere sind Homopolymere oder Copolymere. Erfindungsgemäße Copolymere können dabei neben einer oder mehreren Strukturen gemäß Formel (1) eine oder mehrere weitere Strukturen, beispielsweise aus den oben genannten Gruppen 1 bis 7, besitzen.
Die erfindungsgemäßen Copolymere können statistische, alternierende oder blockartige Strukturen aufweisen oder auch mehrere dieser Strukturen abwechselnd besitzen. Dies kann sich auch auf die Taktizität beziehen. Wie Copolymere mit blockartigen Strukturen erhalten werden können, ist beispielsweise ausführlich in WO 05/014688 beschrieben. Durch das Verwenden verschiedener Strukturelemente können Eigenschaften wie Löslichkeit, Festphasenmorphologie, Farbe, Ladungsinjektions- und -transporteigenschaften, elektrooptische Charakteristik, etc. eingestellt werden. Ebenso können die Polymere linear oder verzweigt aufgebaut sein oder auch dendritische Strukturen enthalten.

Die genaue Struktur des Polymers und die exakte Anordnung der Wiederholeinheiten im Polymer kann entscheidend für die Funktion sein. Ohne an eine bestimmte Theorie gebunden sein zu wollen, vermuten wir, dass in vielen vollständig konjugierten Polymeren entweder die Ladungsmobilität für Löcher und/oder für Elektronen zu hoch ist, so dass sich insgesamt kein ausbalanciertes Ladungsgleichgewicht einstellen kann. Als Folge ist ein Ladungsträger unnötig in zu hohem Anteil vorhanden, was wiederum zu Temperaturerhöhung durch hohe. Ströme und Nebenreaktionen im Polymer oder im Device führen könnte, wodurch die Lebensdauer der Vorrichtung sinkt. Durch einen schlecht ausbalancierten Ladungstransport ist außerdem die Effizienz niedriger, als sie es bei ausgeglichenem Loch- und Elektronentransport sein könnte. Durch Einführung der erfindungsgemäßen Einheiten gemäß Formel (1) und den gezielten Einbau der einzelnen Funktionalitäten in das Polymer kann diesem Problem abgeholfen werden: Durch Reduzierung, jedoch nicht vollständige Unterbrechung der Konjugation und dadurch des Ladungstransports durch die Einheiten gemäß Formel (1) kann die Ladungsträgermobilität für die entsprechenden Ladungsträger gezielt eingestellt und damit die Ladungsbalance in der elektronischen Vorrichtung verbessert werden. Der kontrollierte Aufbau derartiger Strukturen kann beispielsweise durch Kupplung gemäß Suzuki erfolgen, wobei durch die selektive Reaktion von Halogenen mit Boronsäurederivaten die Anordnung der Monomere genau kontrolliert werden kann.
Ein derartiger Aufbau, in dem die emittierenden Abschnitte des Polymers zwischen zwei Einheiten gemäß Formel (1) näherungsweise limitiert sind, hat weiterhin den Vorteil, dass hier die Konjugationslänge definiert ist, was wiederum zu einer weniger breitbandigen Emission und dadurch zu größerer Farbreinheit führt.
Insbesondere für Polymere, die aus dem Triplett-Zustand Licht emittieren, scheint es bevorzugt zu sein, nicht vollständig konjugierte Polymer zu verwenden. Ohne an eine bestimmte Theorie gebunden sein zu wollen, vermuten wir, dass in konjugierten Polymeren der Energieübertrag auf den Triplett-Emitter häufig nicht vollständig ist, bzw. dass ein Rücktransfer vom Triplett-Emitter auf das Polymer stattfinden kann. Dieses Problem kann verringert werden, indem Einheiten gemäß Formel (1) verwendet werden.

Die erfindungsgemäßen Polymere werden in der Regel durch Polymerisation von einer oder mehreren Monomersorten hergestellt, von denen mindestens ein Monomer im Polymer zu Einheiten gemäß Formel (1) führt. Es haben sich hier einige Polymerisationsreaktionen besonders bewährt, die zu C-C- bzw. zu C-N-Verknüpfungen führen:
(A) Polymerisation gemäß SUZUKI;
(B) Polymerisation gemäß YAMAMOTO;
(C) Polymerisation gemäß STILLE;
(D) Polymerisation gemäß HARTWIG-BUCHWALD.
Wie die Polymerisation nach diesen Methoden durchgeführt werden kann und wie die Polymere vom Reaktionsmedium abgetrennt und gereinigt werden können, ist beispielsweise in WO 03/048225 und WO 04/022626 beschrieben. Wie besonders reine Polymere erhalten werden können, ist in der nicht offen gelegten Anmeldung EP 04023475.9 beschrieben.

Monomere, die in erfindungsgemäßen Polymeren zu Struktureinheiten gemäß Formel (1) führen, sind Binaphthyl-Derivate, die in der 2- und/oder 2'-Position geeignet substituiert sind und an der 4,4'-Position (bzw. in einer geeigneten Position an der Gruppe Y, falls vorhanden) geeignete Funktionalitäten aufweisen, die es erlauben, diese Monomereinheit in das Polymer einzubauen. Diese Monomere sind beispielsweise auf folgende Weise zugänglich: Kommerielles 2,2'-Dihydroxy-1,1'-binaphthyl wird in das entsprechende Triflat umgewandelt und dieses in einer Grignard-Kreuzkupplungsreaktion zu den entsprechend substituierten Binaphthylen umgesetzt (Schema 1). Die Produkte können dann regioselektiv an der 4,4'-Position bromiert werden und die Bromide wiederum in die für beispielsweise die Suzuki-Polymerisation benötigten Boronsäuren umgewandelt werden.

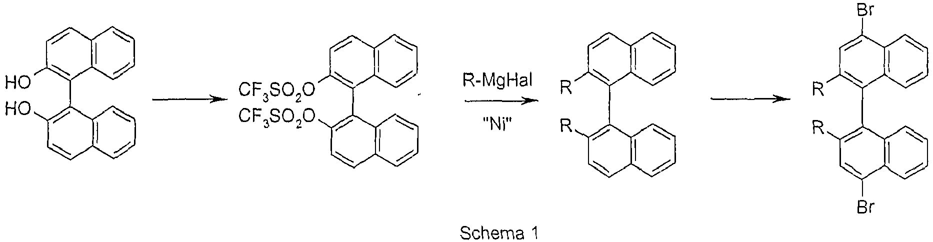

Ein weiterer Zugang ist in Schema 2 gezeigt:

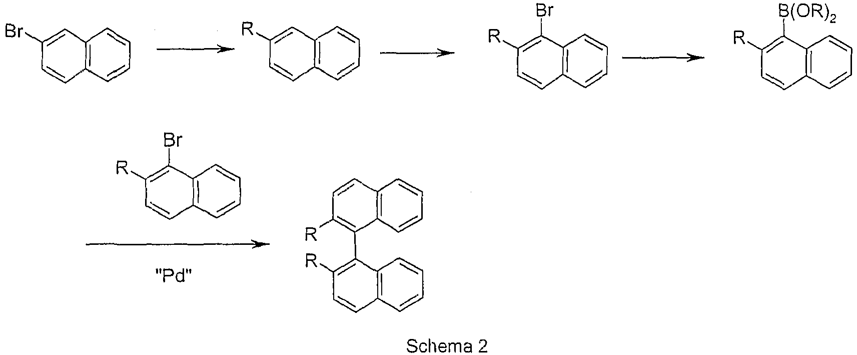

Der etwas größere Syntheseaufwand in Schema 2 wird durch die geringeren Kosten der Rohstoffe und durch die Tatsache, dass auf diesem Wege die asymmetrischen Binaphthyle zugänglich sind, gerechtfertigt. Monomere, die im Polymer zu Einheiten gemäß Formel (1) führen, sind neu.

Weiterhin Gegenstand der Erfindung ist die Verwendung von bifunktionellen monomeren Verbindungen gemäß Formel (2), 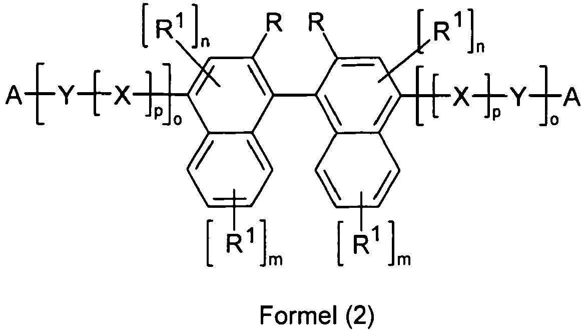 dadurch gekennzeichnet, dass die beiden funktionellen Gruppen A, gleich oder verschieden bei jedem Auftreten, unter Bedingungen der C-C- bzw. C-N-Verknüpfungen durch Polykondensation copolymerisieren und ausgewählt sind aus Cl, Br, I, O-Tosylat, O-Triflat, O-SO₂R², B(OR²)₂ und Sn(R²)₃, bevorzugt aus Br, I und B(OR²)₂, wobei R² dieselbe Bedeutung hat, wie oben beschrieben, und wobei auch zwei oder mehr Reste R² miteinander ein Ringsystem bilden können; die weiteren Symbole und Indizes haben dieselbe Bedeutung wie in Formel (1) definiert.

Nochmals ein weiterer Gegenstand der vorliegenden Erfindung sind bifunktionelle monomere Verbindungen der Formel (2), wie oben abgebildet, mit der Maßgabe, dass die folgenden Verbindungen von der Erfindung ausgenommen sind:

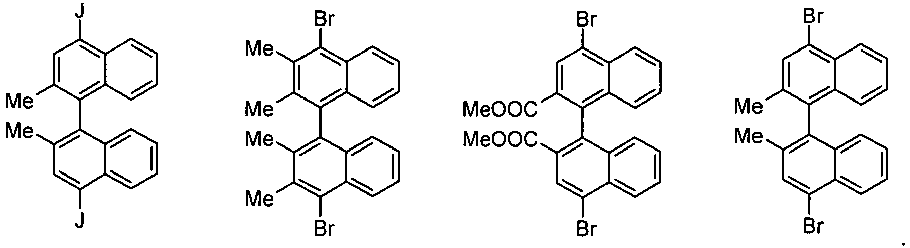

Die C-C-Verknüpfungen sind bevorzugt ausgewählt aus den Gruppen der SUZUKI-Kupplung, der YAMAMOTO-Kupplung und der STILLE-Kupplung; die C-N-Verknüpfung ist bevorzugt eine Kupplung gemäß HARTWIG-BUCHWALD. Besonders bevorzugt ist die SUZUKI-Kupplung.

Dabei gelten für bifunktionelle monomere Verbindungen gemäß Formel (2) dieselben Bevorzugungen wie für die Struktureinheiten gemäß Formel (1).

Die Monomere gemäß Formel (2) liegen in zwei enantiomeren Formen vor. Dabei umfasst die Erfindung sowohl die beiden enantiomerenreinen Formen, wie auch das Racemat und auch Mischungen, in denen eines der beiden Enantiomere in angereicherter Form vorliegt.

Es kann bevorzugt sein, das erfindungsgemäße Polymer nicht als Reinsubstanz, sondern als Mischung (Blend) zusammen mit weiteren polymeren, oligomeren, dendritischen oder niedermolekularen Substanzen zu verwenden. Diese können beispielsweise die elektronischen Eigenschaften verbessern, den Transfer vom Singulett- zum Triplettzustand beeinflussen oder selber Licht emittieren. So ist beispielsweise eine bevorzugte Ausführungsform der Erfindung das Zumischen einer Verbindung, die bei Raumtemperatur aus dem Triplett-Zustand Licht emittieren kann, so dass die Mischung befähigt ist, mit hoher Effizienz aus dem Triplett-Zustand Licht zu emittieren. Derartige Verbindungen sind oben bereits als weitere Strukturelemente für das Polymer beschrieben. Für diese zugemischten Triplett-Emitter gelten dieselben Bevorzugungen, wie oben bereits beschrieben. Auch elektronisch inerte Substanzen können sinnvoll sein, um beispielsweise die Morphologie des gebildeten Polymerfilms oder die Viskosität von Polymerlösungen zu beeinflussen. Solche Mischungen sind daher auch Gegenstand der vorliegenden Erfindung.

Gegenstand der Erfindung sind weiterhin Lösungen und Formulierungen aus einem oder mehreren erfindungsgemäßen Polymeren oder Blends in einem oder mehreren Lösungsmitteln. Wie Polymerlösungen hergestellt werden können, ist beispielsweise in WO 02/072714, in WO 03/019694 und in der darin zitierten Literatur beschrieben.

Die erfindungsgemäßen Polymere können in PLEDs verwendet werden. Diese enthalten Kathode, Anode, Emissionsschicht und gegebenenfalls weitere Schichten, wie z. B. bevorzugt eine Lochinjektionsschicht und gegebenenfalls eine Zwischenschicht zwischen der Lochinjektions- und der Emissionsschicht. Wie PLEDs hergestellt werden können, wird als allgemeines Verfahren ausführlich in WO 04/037887 beschrieben, das entsprechend für den Einzelfall anzupassen ist. Wie oben beschrieben, eignen sich die erfindungsgemäßen Polymere ganz besonders als Elektrolumineszenzmaterialien in derart hergestellten PLEDs oder Displays.

Als Elektrolumineszenzmaterialien im Sinne der Erfindung gelten Materialien, die als aktive Schicht in einer PLED Verwendung finden können. Aktive Schicht bedeutet, dass die Schicht befähigt ist, bei Anlegen eines elektrischen Feldes Licht abzustrahlen (lichtemittierende Schicht) und/oder dass sie die Injektion und/oder den Transport der positiven und/oder negativen Ladungen verbessert (Ladungsinjektions- oder Ladungstransportschicht). Es kann sich auch um eine Zwischenschicht zwischen einer Lochinjektionsschicht und einer Emissionsschicht handeln. Bevorzugt handelt es sich um eine emittierende Schicht.

Gegenstand der Erfindung ist daher auch die Verwendung eines erfindungsgemäßen Polymers oder Blends in einer PLED, insbesondere als Elektrolumineszenzmaterial.

Gegenstand der Erfindung ist ebenfalls eine PLED mit einer oder mehreren aktiven Schichten, wobei mindestens eine dieser aktiven Schichten ein oder mehrere erfindungsgemäße Polymere oder Blends enthält. Die aktive Schicht kann beispielsweise eine lichtemittierende Schicht und/oder eine Transportschicht und/oder eine Ladungsinjektionsschicht und/oder eine Zwischenschicht sein.

Die erfindungsgemäßen Polymere weisen gegenüber den in WO 03/020790 beschriebenen Poly-Spirobifluorenen und Polyfluorenen, beschrieben in WO 02/077060, die hiermit als nächstliegender Stand der Technik genannt werden, folgende überraschenden Vorteile auf:
(1) Es wurde gefunden, dass die erfindungsgemäßen Polymere, die Einheiten gemäß Forme! (1) zusätzlich zu anderen Grundgerüst-Einheiten enthalten, eine deutlich bessere Löslichkeit aufweisen. Dadurch sind Polymere zugänglich, die in einer größeren Bandbreite an Lösemitteln löslich sind, wobei das bislang teilweise nötige, ökologisch bedenkliche und nicht in technischen Prozessierungsschritten verwendbare Chlorbenzol als Lösemittel vermieden werden kann. Als besonderer Vorteil hat sich dabei erwiesen, dass an den Einheiten gemäß Formel (1) keine langen Alkyl- oder Alkoxyketten nötig sind, die nichts zur elektronischen Funktion des Polymers beitragen und die Konzentration der funktionellen Einheiten im Polymer herabsetzen.
(2) Des Weiteren hat sich überraschend gezeigt, dass wiederum im direkten Vergleich die erfindungsgemäßen Polymere in Kombination mit Triplett-Emittern eine höhere Effzienz aufweisen. Dies bezieht sich vor allem auch grüne Triplett-Emitter. Weiterhin erhält man Triplett-Emission mit größerer Farbreinheit, da bei den erfindungsgemäßen Polymeren nur die Emission des Triplett-Emitters, nicht jedoch die Restemission des Polymergrundgerüsts beobachtet wird, was bei Polymeren gemäß dem Stand der Technik teilweise noch der Fall ist.
(3) Die Zugänglichkeit und die Erzielbarkeit von Farben ist bei den erfindungsgemäßen Polymeren gleichwertig oder besser im Vergleich zum Stand der Technik. Insbesondere bei blau emittierenden Polymeren wird ein verbesserter Farbort und eine gesättigtere blaue Emission beobachtet. Auch die Emissionsbanden sind schärfer, was möglicherweise in der reduzierten Konjugation des Polymers begründet liegt
(4) Da das neue Polymergrundgerüst gemäß Formel (1) selber zu tiefblauer Emission führt, ist es leicht möglich, bestimmte emittierende Einheiten einzuführen, die im Polymer zu blauer Emission führen. Dadurch ist es möglich, Ladungstransport- und Emissionseigenschaften im Polymer zu trennen. Wir glauben, dass dies nötig ist, um stabile Polymere zu erhalten. Bislang war dies jedoch nur schwierig möglich, da das Polymergrundgerüst selber immer gleichzeitig auch emittiert hat.

Im vorliegenden Anmeldetext und auch in den im Weiteren folgenden Beispielen wird auf die Verwendung der erfindungsgemäßen Polymere oder Blends in Bezug auf PLEDs und die entsprechenden Displays abgezielt. Trotz dieser Beschränkung der Beschreibung ist es für den Fachmann ohne weiteres erfinderisches Zutun möglich, die erfindungsgemäßen Polymere auch für weitere Verwendungen in anderen elektronischen Devices (Vorrichtungen) zu benutzen, z. B. für organische integrierte Schaltungen (O-ICs), organische Feld-Effekt-Transistoren (O-FETs), organische Dünnfilmtransistoren (O-TFTs), organische Solarzellen (O-SCs), organische Feld-Quench-Devices (O-FQDs), organische lichtemittierende Transistoren (O-LETs) oder auch organische Laserdioden (O-Laser), um nur einige Anwendungen zu nennen.
Die Verwendung der erfindungsgemäßen Polymere in den entsprechenden Vorrichtungen ebenso wie diese Vorrichtungen selbst sind ebenfalls ein Gegenstand der vorliegenden Erfindung.

### Beispiele:

### Beispiel 1: Synthese von Monomeren, die in Polymeren zu Einheiten gemäß Formel (1) führen

### 1.1 Herstellung von 2,2-substituierten 4,4'-verknüpfbaren 1,1'-Binaphthalinen

### 1.1.1 Herstellung von 2-(Pentamethylphenyl)-naphthalin

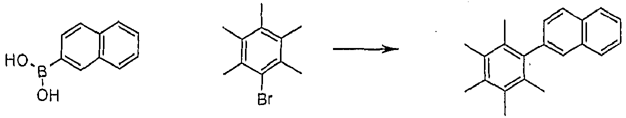

44.3 g (195 mmol) Brompentamethylbenzol, 45.2 g (263 mmol) Naphthalin-2-boronsäure und 120 g (522 mmol) Kaliumphosphat werden in 120 mL Toluol, 120 mL Dioxan und 240 mL Wasser suspendiert und 30 min mit N₂ gesättigt. Anschließend werden 2.96 g (9.71 mmol) o-Tolylphosphin und nach 5 min. Rühren 365 mg (1.62 mmol) Pd(OAc)₂ zugegeben. Die Reaktionsmischung wird 1 h unter Rückfluss erhitzt. Nach dem Abkühlen auf RT wird mit Ethylacetat erweitert, die organische Phase abgetrennt, mit Wasser gewaschen, über Na₂SO₄ getrocknet und einrotiert. Zur weiteren Reinigung wird der Rückstand aus EtOH umkristallisiert. Die Ausbeute beträgt 40 g (75%).

### 1.1.2 Herstellung von 1-Brom-2-(pentamethylphenyl)-naphthalin

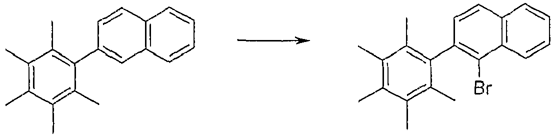

46.1 g (168 mmol) 2-(Pentamethylphenyl)naphthalin werden in 350 mL CHCl₃ gelöst und auf 5 °C abgekühlt. Eine Lösung von 8.6 mL (168 mmol) Brom in 40 mL CHCl₃ wird innerhalb 1 h bei dieser Temperatur zugetropft. Nach 1 h werden 50 mL gesättigte Na₂SO₃-Lsg. zugetropft, die org. Phase abgetrennt, zweimal mit Wasser gewaschen, über Na₂SO₄ getrocknet und das Lösungsmittel i. V. entfernt. Nach Umkristallisation aus EtOH/Toluol erhält man das Bromid in Form farbloser Kristalle (52.2 g, 88%).

### 1.1.3 Herstellung von 2-Pentamethylphenyl-1-(1-naphthyl)-naphthalin

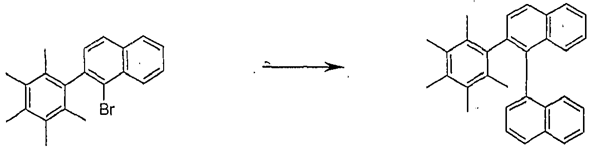

10.2 g (59 mmol) 1-Naphthylboronsäure, 10.4 g (29.4 mmol) 1-Brom-2-(pentamethylphenyl)naphthalin und 29.9 g (130 mmol) Kaliumphosphat werden in 35 mL Toluol, 35 mL Dioxan und 70 mL Wasser suspendiert und 30 min mit N₂ gesättigt. Anschließend werden 550 mg (1.8 mmol) o-Tolylphosphin und nach 5 min. Rühren 67 mg (0.3 mmol) Pd(OAc)₂ zugegeben. Die Reaktionsmischung wird 2 h unter Rückfluss erhitzt. Nach dem Abkühlen auf RT wird mit Ethylacetat erweitert, die org. Phase abgetrennt, dreimal mit Wasser gewaschen, über Na₂SO₄ getrocknet und i. V. vom Lösungsmittel befreit. Das verbliebene Öl wird aus i-PrOH kristalliert. 10 g (82%) Ausbeute in Form eines farblosen Pulvers.

### 1.1.4 Herstellung von 4,4'-Dibrom-2-pentamethylphenyl-1-(1-naphthyl)-naphthalin (M1)

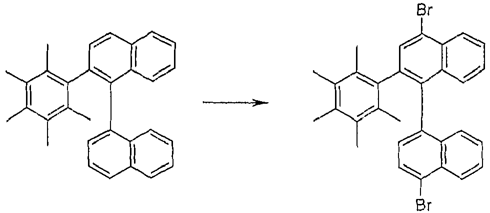

9 g (22 mmol) 2-Pentamethylphenyl-1-(1-naphthyl)-naphthalin werden in 100 mL CHCl₃ gelöst und auf 5 °C abgekühlt. Eine Lösung von 2.2 mL (44 mmol) Brom in 10 mL CHCl₃ wird innerhalb 1 h bei dieser Temperatur zugetropft. Nach 1 h werden 20 mL gesättigte Na₂SO₃-Lsg. zugetropft, die org. Phase abgetrennt, zweimal mit Wasser gewaschen, über Na₂SO₄ getrocknet und das Lösungsmittel i. V. entfernt. Nach dreifacher Umkristallisation aus EtOH/Toluol erhält man das Bromid in Form farbloser Kristalle mit einer HPLC-Reinheit von >99.8% (8.3 g, 66%).

### 1.1.5 Herstellung von 4,4'-(2-[1,3,2]dioxaborolanyl)2-pentamethylphenyl-1-(1-naphthyl)-naphthalin (M2)

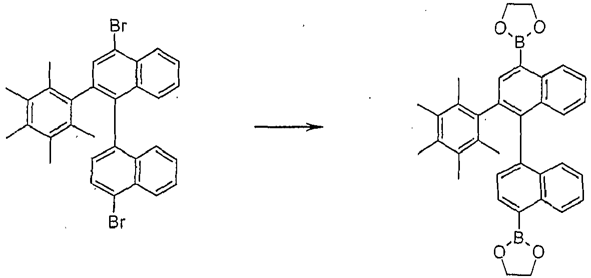

5.8 g Magnesium (235 mmol) werden in einer getrockneten Appartur vorgelegt und aus 67.7 g (112 mmol) Dibromid (aus Beispiel 1.1.4) in 340 mL THF unter Rückfluss das Grignardreagenz hergestellt. Nach 3 h wird die Lösung abgekühlt und zur Lösung von 38 mL (336 mmol) Borsäuretrimethylester in 250 mL THF bei -75 °C getropft. Die Reaktionsmischung wird über einen Zeitraum von 6 h auf RT gebracht, 100 mL Ethylacetat, 30 mL Eisessig und 60 mL H₂O zugegeben, die org. Phase abgetrennt, zweimal mit Wasser gewaschen und über Na₂SO₄ getrocknet. Der nach dem Entfernen des Lösungsmittels verbleibende Feststoff wird in 200 mL Toluol suspendiert, 12.5 mL wasserfreies Ethylenglycol zugegeben und die Suspension am Wasserabscheider für 4 h zum starken Sieden erhitzt. Nach erneutem Entfernen des Lösungsmittels wird der verbliebene Feststoff viermal aus Acetonitril umkristallisiert. Man erhält den Diester als farblose Kristalle mit einer Reinheit von >99.9%, die Ausbeute beträgt 36 g (58%).

### 1.1.6 Herstellung von 4,4'-Dibrom-2,2'-dimethyl-1,1'-binaphthyl (M3)

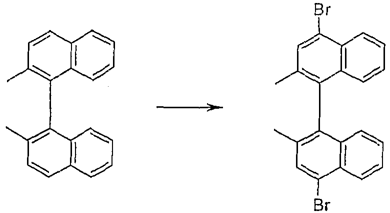

59.2 g (200 mmol) 2,2'-Dimethylbinaphthyl (hergestellt analog N. Maigrot, J. P. Mazaleyrat, Synthesis 1958, 317) werden in 1000 mL CHCl₃ gelöst und auf 5 °C abgekühlt. Eine Lösung von 20 mL (400 mmol) Brom in 90 mL CHCl₃ wird innerhalb 1 h bei dieser Temperatur zugetropft. Nach 1 h werden 180 mL gesättigte Na₂SO₃-Lsg. zugetropft, die org. Phase abgetrennt, zweimal mit Wasser gewaschen, über Na₂SO₄ getrocknet und das Lösungsmittel i. V. entfernt. Nach mehrfacher Umkristallisation aus i-PrOH erhält man das Bromid in Form farbloser Kristalle mit einer HPLC-Reinheit von >99.7% (50.9 g, 56%).

### 1.1.7 Herstellung von 4,4'-(2-[1,3,2]dioxaborolanyl)-2,2'-dimethyl-1,1'-binaphthyl (M4)

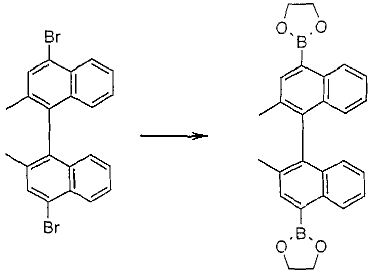

5.8 g Magnesium (235 mmol) werden in einer getrockneten Appartur vorgelegt und aus 50.8 g (112 mmol) Dibromid (aus Beispiel 1.1.6) in 340 mL THF unter Rückfluss das Grignardreagenz hergestellt. Nach 3 h wird die Lösung abgekühlt und zur Lösung von 38 mL (336 mmol) Borsäuretrimethytester in 250 mL THF bei -75 °C getropft. Die Reaktionsmischung wird über einen Zeitraum von 6 h auf RT gebracht, 100 mL Ethylacetat, 30 mL Eisessig und 60 mL H₂O zugegeben, die org. Phase abgetrennt, zweimal mit Wasser gewaschen und über Na₂SO₄ getrocknet. Der nach dem Entfernen des Lösungsmittels verbleibende Feststoff wird in 200 mL Toluol suspendiert, 12.5 mL wasserfreies Ethylenglycol zugegeben und die Suspension am Wasserabscheider für 4 h zum starken Sieden erhitzt. Nach erneutem Entfernen des Lösungsmittels wird der verbliebene Feststoff viermal aus Acetonitril umkristallisiert. Man erhält den Diester als farblose Kristalle mit einer Reinheit von >99.8%, die Ausbeute beträgt 67.5 g (62%).

### 1.1.8 Herstellung von 2,2'-Dioctyl-1,1'-binaphthyl

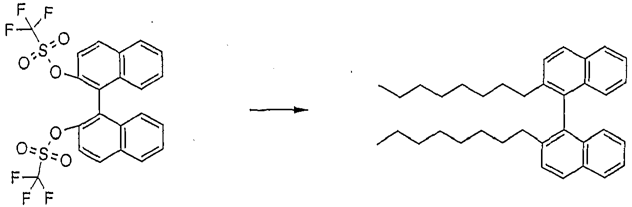

Das Grignardreagenz, hergestellt aus 70 g (364 mmol) Octylbromid und 8.85 g (364 mmol) Magnesium in 350 mL THF wird bei RT zu 50 g (91 mmol) 2,2'-Bis-(trifluormethansulfonyl)-1,1'-binaphthyl getropft, 4.88 g (9.1 mmol) NiCl₂dppp zugegeben und das Gemisch 24 h unter Rückfluss erhitzt. Dann werden 20 mL Essigsäure unter Eiskühlung zugetropft, der Ansatz extraktiv mit Heptan und Wasser aufgearbeitet und die organische Phase über Na₂SO₄ getrocknet. Nach Säulenfiltration an Kieselgel mit Heptan wird der nach Entfernen des Lösungsmittels verbliebene ölige Rückstand mittels Kurzwegdestillation gereinigt. Man erhält 38.3 g (88%) eines gelblichen, hochviskosen Öles.

### 1.1.9 Herstellung von 4,4'-Dibrom-2,2'-dioctyl-1,1'-binaphthyl (M5)

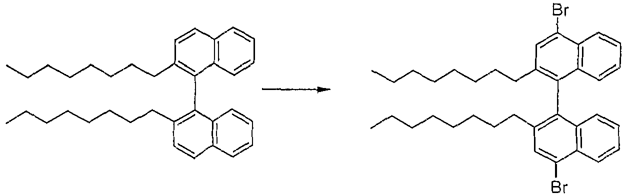

38 g (79 mmol) 2,2'-Dioctyl-1,1'-binaphthyl werden in 400 mL CHCl₃ gelöst und auf 5 °C abgekühlt. Eine Lösung von 8 mL (160 mmol) Brom in 35 mL CHCl₃ wird innerhalb 1 h bei dieser Temperatur zugetropft. Nach 1 h werden 75 mL gesättigte Na₂SO₃-Lsg. zugetropft, die org. Phase abgetrennt, zweimal mit Wasser gewaschen, über Na₂SO₄ getrocknet und das Lösungsmittel i. V. entfernt. Der verbliebene Rückstand wird mittels präparativer HPLC gereinigt. Das Dibromid erhält man danach in Form eines farblosen, zähen Öles mit einer Reinheit von >99.8% und einer Ausbeute von 38 g (76%).

### 1.1.10 Herstellung von 4,4'-(2-[1,3,2]dioxaborolanyl)-2,2'-dioctyl-1,1'-binaphthyl (M6)

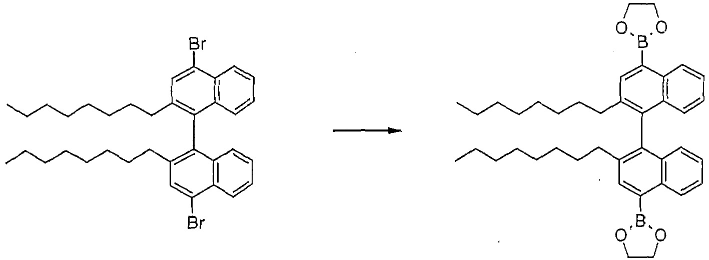

2.9 g Magnesium (118 mmol) werden in einer getrockneten Appartur vorgelegt und aus 35.7 g (56 mmol) Dibromid (aus Beispiel 1.1.9) in 170 mL THF unter Rückfluss das Grignardreagenz hergestellt. Nach 3 h wird die Lösung abgekühlt und zur Lösung von 19 mL (168 mmol) Borsäuretrimethylester in 125 mL THF bei -75 °C getropft. Die Reaktionsmischung wird über einen Zeitraum von 6 h auf RT gebracht, 50 mL Ethylacetat, 15 mL Eisessig und 30 mL H₂O zugegeben, die org. Phase abgetrennt, zweimal mit Wasser gewaschen und über Na₂SO₄ getrocknet. Der nach dem Entfernen des Lösungsmittels verbleibende Feststoff wird in 100 mL Toluol suspendiert, 6.5 mL wasserfreies Ethylenglycol zugegeben und die Lösung am Wasserabscheider für 4 h zum starken Sieden erhitzt. Nach erneutem Entfernen des Lösungsmittels wird der verbliebene Feststoff einmal aus Acetonitril ausgekocht und dreimal aus Pentan umkristalliert. Man erhält den Diester als farblose Kristalle mit einer Reinheit von >99.6%, die Ausbeute beträgt 25.6 g (74%).

### Beispiel 2: Synthese weiterer Monomere

Die Synthese der Monomere **M7** bis **M10** ist in WO 03/020790 und der darin zitierten Literatur beschrieben.

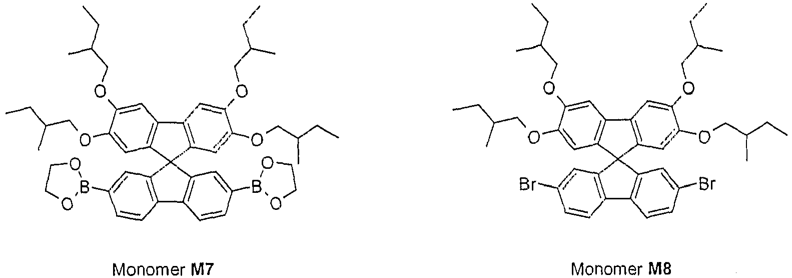

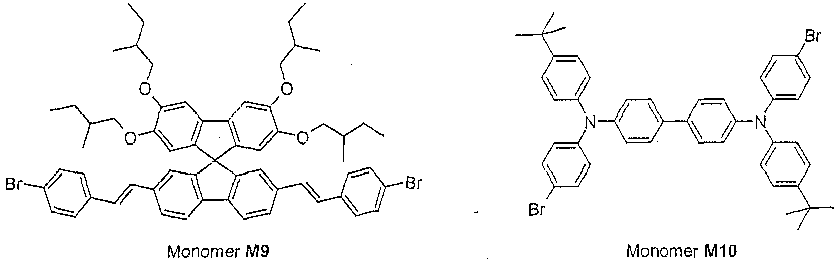

### Beispiel 3: Synthese der Polymere

Die Polymere wurden durch SUZUKI-Kupplung gemäß WO 03/048225 synthetisiert. Die Zusammensetzung der synthetisierten Polymere **P1** bis **P5** ist in Tabelle 1 zusammengefasst. Außerdem wurden die Vergleichspolymere **V1** und **V2** synthetisiert, die statt des Monomers **M1-6,** das im Polymer zu Einheiten gemäß Formel (1) führt, das Monomer **M8** oder/und **M7** enthalten. Die Zusammensetzung dieser Vergleichspolymere ist ebenfalls in Tabelle 1 aufgeführt.

### Beispiel 4: Herstellung der PLEDs

Die Polymere wurden für einen Einsatz in PLEDs untersucht. Die PLEDs waren jeweils Zweischichtsysteme, d. h. Substrat//ITO//PEDOT//Polymer//Kathode. PEDOT ist ein Polythiophen-Derivat (Baytron P, von H. C. Starck, Goslar). Als Kathode wurde in allen Fällen Ba/Ag (Aldrich) verwendet. Wie PLEDs dargestellt werden können, ist in WO 04/037887 und der darin zitierten Literatur ausführlich beschrieben.

### Beispiel 5 bis 11: Device-Beispiele

Die Ergebnisse, die bei Verwendung der Polymere **P1** bis **P5** in PLEDs erhalten wurden, sind in Tabelle 1 zusammengefasst. Ebenso aufgeführt sind die Elektrolumineszenz-Ergebnisse, die unter Verwendung der Vergleichspolymere **V1** bis **V2** erhalten wurden.

Wie man aus den Ergebnissen erkennen kann, ist die Emissionsfarbe der erfindungsgemäßen Polymere tiefer Blau verschoben, und die Lebensdauern sind, insbesondere bezogen auf die Emissionsfarbe, deutlich verbessert. Dies zeigt, dass die erfindungsgemäßen Polymere besser für den Einsatz in Displays geeignet sind als Polymere gemäß dem Stand der Technik.

**Tabelle 1: Device-Ergebnisse mit erfindungsgemäßen Polymeren und Vergleichspolymeren**

| Beispiel | Polymer | Monomer für Einheiten gemäß Formel (1) | Weitere Monomere | Max. Eff. / cd/A | U@ 100 cd/m² /V | CIE x/y^{a} | Lebensdauer^{b} / h |
|---|---|---|---|---|---|---|---|
| 5 | **P1** | 10% **M3** | 50% **M7,** 30% **M8,** 10% **M10** | 2,70 | 4,6 | 0,15/0,15 | 100 |
| 6 | **P2** | 10% **M3** | 50% **M7,** 20% **M8,** 10% **M10,** 10% **M9** | 4,50 | 4,0 | 0,16/0,27 | 610 |
| 7 | **P3** | 50% **M6** | 30% **M8,** 10% **M10,** 10% **M9** | 2,64 | 5,1 | 0,15/0,18 | 400 |
| 8 | **P4** | 50% **M2,** 40% **M5** | 10% **M10** | 1,91 | 5,5 | 0,14/0,12 | 78 |
| 9 | **P5** | 50% **M2,** 30% **M5** | 10% **M10,** 10% **M9** | 2,32 | 4,8 | 0,14/0,16 | 354 |
| 10 (Vergleich) | **V1** | --- | 50% **M7,** 40% **M8,** 10% **M10** | 2,86 | 4,4 | 0,16/0,18 | 80 |
| 11 (Vergleich) | **V2** | --- | 50% **M7,** 30% **M8,** 10% **M10**, 10 % **M9** | 4,66 | 3,9 | 0,17/0,30 | 530 |

| | | | | | | | |
|---|---|---|---|---|---|---|---|
| ^{a} CIE-Koordinaten: Farbkoordinaten der Commision Internationale de l'Eclairage 1931. ^{b} Lebensdauer: Zeit bis zum Abfall der Helligkeit auf 50 % der Anfangshelligkeit, Anfangshelligkeit 400 cd/m². | | | | | | | |

## Patentansprüche

1. **Polymere, enthaltend mindestens 1 mol% einer ersten Wiederholeinheit gemäß Formel (1),** 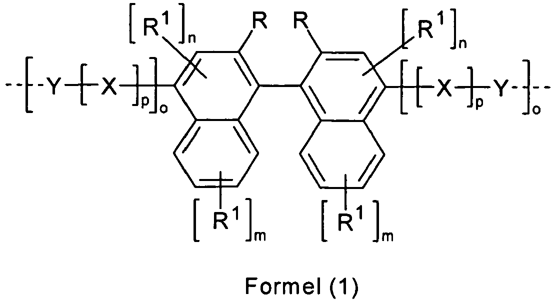 wobei die verwendeten Symbole und Indizes folgende Bedeutung besitzen:
R ist bei jedem Auftreten gleich oder verschieden H, eine geradkettige Alkylkette mit 1 bis 40 C-Atomen oder eine verzweigte oder cyclische Alkylkette mit 3 bis 40 C-Atomen, die jeweils durch R¹ substituiert sein kann und in der auch ein oder mehrere nicht benachbarte C-Atome durch N-R¹, O, S, O-CO-O, CO-O, -CR¹=CR¹- oder -C≡C- ersetzt sein können, mit der Maßgabe, dass die Heteroatome nicht direkt an die Naphthyl-Einheit gebunden sind, und in der auch ein oder mehrere H-Atome durch F, Cl, Br, I oder CN ersetzt sein können, oder ein aromatisches oder heteroaromatisches Ringsystem mit 5 bis 40 aromatischen Ringatomen, welches auch durch einen oder mehrere Reste R¹ substituiert sein kann; dabei können die beiden Reste R miteinander auch ein weiteres Ringsystem bilden; mit der Maßgabe, dass mindestens einer der beiden Reste R ungleich H ist;
X ist bei jedem Auftreten gleich oder verschieden -CR¹=CR¹-, -C≡C- oder N-Ar;
Y ist bei jedem Auftreten gleich oder verschieden ein bivalentes aromatisches oder heteroaromatisches Ringsystem mit 5 bis 40 aromatischen Ringatomen, welches durch einen oder mehrere Reste R¹ substituiert oder unsubstituiert sein kann;
R¹ ist bei jedem Auftreten gleich oder verschieden H, F, Cl, Br, I, CN, N(R²)₂, Si(R²)₃, B(R²)₂, eine geradkettige Alkyl- oder Alkoxykette mit 1 bis 40 C-Atomen oder eine verzweigte oder cyclische Alkyl- oder Alkoxykette mit 3 bis 40 C-Atomen, in der jeweils auch ein oder mehrere nicht benachbarte C-Atome durch N-R², O, S, O-CO-O, CO-O, -CR¹=CR¹- oder -C≡C- ersetzt sein können und in der auch ein oder mehrere H-Atome durch F, Cl, Br, I oder CN ersetzt sein können, oder eine Aryl-, Aryloxy- oder Heteroarylgruppe mit 5 bis 40 aromatischen Ringatomen, welche auch durch einen oder mehrere nicht-aromatische Reste R¹ substituiert sein kann; dabei können auch zwei oder mehrere der Reste R¹ miteinander ein Ringsystem bilden;
R² ist bei jedem Auftreten gleich oder verschieden H oder ein aliphatischer oder aromatischer Kohlenwasserstoffrest mit 1 bis 20 C-Atomen;
Ar ist bei jedem Auftreten gleich oder verschieden ein monovalentes aromatisches oder heteroaromatisches Ringsystem mit 5 bis 40 aromatischen Ringatomen, welches mit R¹ substituiert oder unsubstituiert sein kann;
n ist bei jedem Auftreten gleich oder verschieden 0 oder 1;
m ist bei jedem Auftreten gleich oder verschieden 0, 1, 2, 3 oder 4;
o ist bei jedem Auftreten gleich oder verschieden 0, 1 oder 2;
p ist bei jedem Auftreten gleich oder verschieden 0 oder 1;
die gestrichelte Bindung bedeutet dabei die Verknüpfung im Polymer; und außerdem enthaltend mindestens 1 mol% einer zweiten Wiederholeinheit, die entweder gleich einer Wiederholeinheit gemäß Formel (1) ist oder verschieden ist.

2. Polymere gemäß Anspruch 1, **dadurch gekennzeichnet, dass** es sich um konjugierte oder teilkonjugierte Polymere handelt.

3. Polymere gemäß Anspruch 1 oder 2, **dadurch gekennzeichnet, dass** für Einheiten gemäß Formel (1) gilt:
R ist bei jedem Auftreten gleich oder verschieden H, eine geradkettige Alkylkette mit 1 bis 10 C-Atomen oder eine verzweigte oder cyclische Alkylkette mit 3 bis 10 C-Atomen, in der auch ein oder mehrere nicht benachbarte C-Atome durch -CH=CH- oder -C≡C- ersetzt sein können und in der auch ein oder mehrere H-Atome durch F oder CN ersetzt sein können, oder ein aromatisches oder heteroaromatisches Ringsystem mit 5 bis 20 aromatischen Ringatomen, welches auch durch einen oder mehrere nicht-aromatische Reste R¹ substituiert sein kann; dabei können die beiden Reste R zusammen auch ein weiteres Ringsystem bilden; mit der Maßgabe, dass mindestens ein Rest R ungleich H ist;
Y ist bei jedem Auftreten gleich oder verschieden eine bivalente Aryl- oder Heteroarylgruppe mit 5 bis 20 aromatischen Ringatomen, welche durch einen oder mehrere Reste R¹ substituiert sein kann;
Ar ist bei jedem Auftreten gleich oder verschieden eine monovalente Aryl- oder Heteroarylgruppe mit 5 bis 20 aromatischen Ringatomen, welche mit R¹ substituiert oder unsubstituiert sein kann;
R¹ ist bei jedem Auftreten gleich oder verschieden H, F, N(R²)₂, eine geradkettige Alkylkette mit 1 bis 10 C-Atomen oder eine verzweigte Alkylkette mit 3 bis 10 C-Atomen, in der jeweils auch ein oder mehrere nicht benachbarte C-Atome durch -CR¹=CR¹- oder -C≡C- ersetzt sein können und in der auch ein oder mehrere H-Atome durch F ersetzt sein können, oder eine Aryl- oder Heteroarylgruppe mit 5 bis 20 aromatischen Ringatomen, welche auch durch einen oder mehrere nicht-aromatische Reste R¹ substituiert sein kann; dabei können auch zwei oder mehrere der Reste R¹ miteinander ein Ringsystem bilden;
n ist bei jedem Auftreten gleich 0;
m ist bei jedem Auftreten gleich oder verschieden 0, 1 oder 2;
o ist bei jedem Auftreten gleich oder verschieden 0 oder 1;
die weiteren Symbole und Indizes sind wie unter Anspruch 1 definiert.

4. Polymere gemäß einem oder mehreren der Ansprüche 1 bis 3, **dadurch gekennzeichnet, dass** die beiden Reste R gleich sind.

5. Polymere gemäß einem oder mehreren der Ansprüche 1 bis 3, **dadurch gekennzeichnet, dass** einer der beiden Reste R gleich H und der andere ein aromatisches oder heteroaromatisches Ringsystem mit 5 bis 15 aromatischen Ringatomen ist.

6. Polymere gemäß einem oder mehreren der Ansprüche 1 bis 5, **dadurch gekennzeichnet, dass** das Polymer weitere Strukturelemente enthält, ausgewählt aus der Gruppe der Fluoren-Derivate, der Spirobifluoren-Derivate, der Dihydrophenanthren-Derivate, der cis- oder trans-Indenofluoren-Derivate, der 1,4-Phenylen-Derivate, der 4,4'-Biphenylylen-Derivate, der 4,4"-Terphenylylen-Derivate, der 2,7- oder 3,6-Phenanthren-Derivate, der Dihydropyren- oder Tetrahydropyren-Derivate, die durch R¹ substituiert oder unsubstituiert sein können.

7. Polymere gemäß einem oder mehreren der Ansprüche 1 bis 6, **dadurch gekennzeichnet, dass** das Polymere weitere Strukturelemente enthält, ausgewählt aus der Gruppe der durch R¹ substituierten oder unsubstituierten kondensierten aromatischen Strukturen mit 6 bis 40 C-Atomen oder Tolan-, Stilben- oder Bisstyrylarylenderivaten.

8. Polymere gemäß einem oder mehreren der Ansprüche 1 bis 7, **dadurch gekennzeichnet, dass** das Polymer weitere Strukturelemente enthält, ausgewählt aus der Gruppe der durch R¹ substituierten oder unsubstituierten Triarylamine, Benzidine, N,N,N',N'-Tetraaryl-para-phenylendiamine, Triarylphosphine, Phenothiazine, Phenoxazine, Dihydrophenazine, Thianthrene, Dibenzo-p-dioxine, Phenoxathiine, Carbazole, Azulene, Thiophene, Pyrrole, Furane und weiterer O-, S- oder N-haltigen Heterocyclen mit hoch liegendem HOMO.

9. Polymere gemäß einem oder mehreren der Ansprüche 1 bis 8, **dadurch gekennzeichnet, dass** das Polymer weitere Strukturelemente enthält, ausgewählt aus der Gruppe der durch R¹ substituierten oder unsubstituierten Pyridine, Pyrimidine, Pyridazine, Pyrazine, Triazine, Oxadiazole, Chinoline, Chinoxaline oder Phenazine, der Triarylborane und weiterer O-, S- oder N-haltiger Heterocyclen mit niedrig liegendem LUMO.

10. Polymere gemäß einem oder mehreren der Ansprüche 1 bis 9, **dadurch gekennzeichnet, dass** das Polymer Strukturelemente gemäß Anspruch 8 und gemäß Anspruch 9 enthält, die direkt aneinander gebunden sind.

11. Polymere gemäß einem oder mehreren der Ansprüche 1 bis 10, **dadurch gekennzeichnet, dass** weitere Strukturelemente anwesend sind, die bei Raumtemperatur mit hoher Effizienz aus dem Triplettzustand Licht emittieren und Elektrophosphoreszenz statt Elektrofluoreszenz zeigen und die mindestens ein Schweratom mit einer Ordnungszahl von mehr als 36 enthalten.

12. Polymere gemäß Anspruch 11, **dadurch gekennzeichnet, dass** zusätzlich zu dem Triplett-Emitter Strukturelemente anwesend sind, ausgewählt aus der Gruppe der Carbazole, der überbrückten Carbazol-Dimere, der Ketone, Phosphinoxide, Sulfoxide, Sulfone oder der Silane.

13. Polymere gemäß einem oder mehreren der Ansprüche 1 bis 12, **dadurch gekennzeichnet, dass** der Anteil Einheiten gemäß Formel (1) mindestens 5 mol% beträgt.

14. Polymer gemäß einem oder mehreren der Ansprüche 1 bis 13, **dadurch gekennzeichnet, dass** die Einheit gemäß Formel (1) als Polymer-Grundgerüst bzw. in Kombination mit einem Polymer-Grundgerüst eingesetzt wird und dass gilt:
p ist bei jedem Auftreten gleich 0.

15. Polymer gemäß einem oder mehreren der Ansprüche 1 bis 13, dadurch gekennzeichent, dass die Einheit gemäß Formel (1) als lochtransportierende Einheit eingesetzt wird und dass gilt:
p ist bei jedem Auftreten gleich oder verschieden 0 oder 1, wobei mindestens ein p = 1 ist;
o ist bei jedem Aufteten gleich oder verschieden 0, 1 oder 2, wobei o ungleich 0 ist, wenn das entsprechende p = 1 ist;
X ist bei jedem Auftreten N-Ar.

16. Polymer gemäß einem oder mehreren der Ansprüche 1 bis 13, **dadurch gekennzeichnet, dass** die Einheit gemäß Formel (1) als Emitter eingesetzt wird und dass gilt:
p ist bei jedem Auftreten gleich oder verschieden 0 oder 1, wobei mindestens ein p = 1 ist;
o ist bei jedem Auftreten gleich oder verschieden 0, 1 oder 2, wobei o ungleich 0 ist, wenn das entsprechende p = 1 ist;
X ist bei jedem Auftreten gleich oder verschieden -CR¹=CR¹-, -C≡C- oder N-Ar, wobei mindestens ein X gleich -CR¹=CR¹- oder -C≡C- ist.

17. Verwendung von bifunktionellen monomeren Verbindungen gemäß Formel (2), 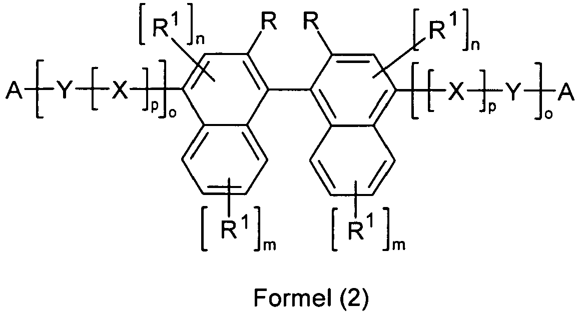 **dadurch gekennzeichnet, dass** die beiden funktionellen Gruppen A, gleich oder verschieden bei jedem Auftreten, unter Bedingungen der C-C- bzw. C-N-Verknüpfungen copolymerisieren und ausgewählt sind aus Cl, Br, I, O-Tosylat, O-Triflat, O-SO₂R², B(OR²)₂ und Sn(R²)₃, wobei R² dieselbe Bedeutung hat, wie in Anspruch 1 beschrieben, und wobei auch zwei oder mehr Reste R² miteinander ein Ringsystem bilden können; die weiteren Symbole und Indizes haben dieselbe Bedeutung wie in Anspruch 1 beschrieben zur Herstellung von Polymeren.

18. Bifunktionelle monomere Verbindungen gemäß Formel (2) gemäß Anspruch 17, mit der Maßgabe, dass die folgenden Verbindungen von der Erfindung ausgenommen sind:

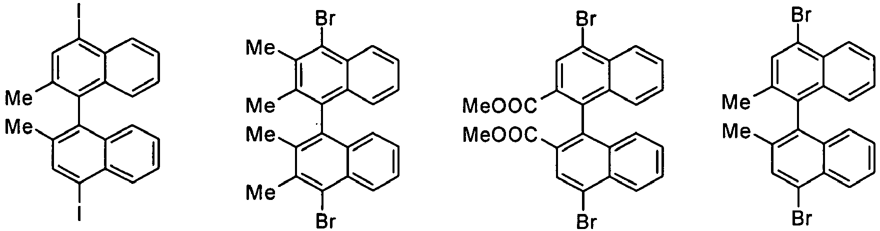

19. Mischungen (Blends) aus einem oder mehreren Polymeren gemäß einem oder mehreren der Ansprüche 1 bis 16 mit weiteren polymeren, oligomeren, dendritischen oder niedermolekularen Substanzen.

20. Mischungen gemäß Anspruch 19, **dadurch gekennzeichnet, dass** eine Verbindung zugemischt wird, die bei Raumtemperatur aus dem Triplett-Zustand Licht emittieren kann.

21. Lösungen und Formulierungen aus einem oder mehreren Polymeren oder Blends gemäß einem oder mehreren der Ansprüche 1 bis 16 und/oder 19 bis 20 in einem oder mehreren Lösungsmitteln.

22. Verwendung eines Polymers oder Blends oder einer Lösung gemäß einem oder mehreren der Ansprüche 1 bis 16 und/oder 19 bis 21 in einer organischen elektronischen Vorrichtung.

23. Organische elektronische Vorrichtung mit einer oder mehreren aktiven Schichten, wobei mindestens eine dieser aktiven Schichten ein oder mehrere Polymere oder Blend gemäß einem oder mehreren der Ansprüche 1 bis 16 und/oder 19 bis 20 enthält.

24. Organische elektronische Vorrichtung gemäß Anspruch 23, ausgewählt aus der Gruppe der polymeren Leuchtdioden (PLEDs), organischen integrierten Schaltungen (O-ICs), organischen Feld-Effekt-Transistoren (O-FETs), organischen Dünnfilmtransistoren (O-TFTs), organischen Solarzellen (O-SCs), organischen Feld-Quench-Vorrichtungen (O-FQDs), organischen lichtemittierenden Transistoren (O-LETs) oder organischen Laserdioden (O-Laser).

## Claims

1. Polymers containing at least 1 mol% of a first recurring unit of the formula (1), 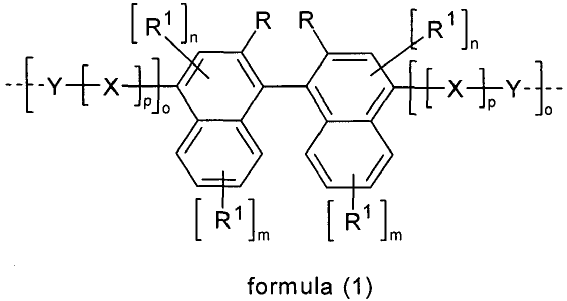 where the symbols and indices used have the following meaning:
R is on each occurence, identically or differently, H, a straight-chain alkyl chain having 1 to 40 C atoms or a branched or cyclic alkyl chain having 3 to 40 C atoms, each of which may be substituted by R¹ and in which, in addition, one or more non-adjacent C atoms may be replaced by N-R¹, O, S, O-CO-O, CO-O, -CR¹=CR¹- or -C≡C-, with the proviso that the heteroatoms are not bonded directly to the naphthyl unit, and in which, in addition, one or more H atoms may be replaced by F, Cl, Br, I or CN, or an aromatic or heteroaromatic ring system having 5 to 40 aromatic ring atoms, which may also be substituted by one or more radicals R¹; the two radicals R here may also form a further ring system with one another; with the proviso that at least one of the two radicals R is not equal to H;
X is on each occurence, identically or differently, -CR¹=CR¹-, -C≡C- or N-Ar;
Y is on each occurence, identically or differently, a divalent aromatic or heteroaromatic ring system having 5 to 40 aromatic ring atoms, which may be substituted by one or more radicals R¹ or unsubstituted;
R¹ is on each occurence, identically or differently, H, F, Cl, Br, I, CN, N(R²)₂, Si(R²)₃, B(R²)₂, a straight-chain alkyl or alkoxy chain having 1 to 40 C atoms or a branched or cyclic alkyl or alkoxy chain having 3 to 40 C atoms, in which, in addition, one or more non-adjacent C atoms may be replaced by N-R², O, S, O-CO-O, CO-O, -CR¹=CR¹- or -C≡C- and in which, in addition, one or more H atoms may be replaced by F, Cl, Br, I or CN, or an aryl, aryloxy or heteroaryl group having 5 to 40 aromatic ring atoms, which may also be substituted by one or more non-aromatic radicals R¹; two or more of the radicals R¹ here may also form a ring system with one another;
R² is on each occurence, identically or differently, H or an aliphatic or aromatic hydrocarbon radical having 1 to 20 C atoms;
Ar is on each occurence, identically or differently, a monovalent aromatic or heteroaromatic ring system having 5 to 40 aromatic ring atoms, which may be substituted by R¹ or unsubstituted;
n is on each occurence, identically or differently, 0 or 1;
m is on each occurence, identically or differently, 0, 1, 2, 3 or 4;
o is on each occurence, identically or differently, 0, 1 or 2;
p is on each occurence, identically or differently, 0 or 1;
the dashed bond here denotes the linking in the polymer;
and additionally containing at least 1 mol% of a second recurring unit, which is either identical to or different from a recurring unit of the formula (1).

2. Polymers according to Claim 1, **characterised in that** they are conjugated or part-conjugated polymers.

3. Polymers according to Claim 1 or 2, **characterised in that**, for units of the formula (1):
R is on each occurence, identically or differently, H, a straight-chain alkyl chain having 1 to 10 C atoms or a branched or cyclic alkyl chain having 3 to 10 C atoms, in which, in addition, one or more non-adjacent C atoms may be replaced by -CH=CH- or -C≡C- and in which, in addition, one or more H atoms also be replaced by F or CN, or an aromatic or heteroaromatic ring system having 5 to 20 aromatic ring atoms, which may also be substituted by one or more non-aromatic radicals R¹; the two radicals R here may also form a further ring system with one another, with the proviso that at least one radical R is not equal to H;
Y is on each occurence, identically or differently, a divalent aryl or heteroaryl group having 5 to 20 aromatic ring atoms, which may be substituted by one or more radicals R¹;
Ar is on each occurence, identically or differently, a monovalent aryl or heteroaryl group having 5 to 20 aromatic ring atoms, which may be substituted by R¹ or unsubstituted;
R¹ is on each occurence, identically or differently, H, F, N(R²)₂, a straightchain alkyl chain having 1 to 10 C atoms or a branched alkyl chain having 3 to 10 C atoms, in which, in addition, one or more non-adjacent C atoms may be replaced by -CR¹=CR¹- or -C≡C- and in which, in addition, one or more H atoms may be replaced by F, or an aryl or heteroaryl group having 5 to 20 aromatic ring atoms, which may also be substituted by one or more non-aromatic radicals R¹; two or more of the radicals R¹ here may also form a ring system with one another;
n is on each occurence equal to 0;
m is on each occurence, identically or differently, 0, 1 or 2;
o is on each occurence, identically or differently, 0 or 1;
the further symbols and indices are as defined under Claim 1.

4. Polymers according to one or more of Claims 1 to 3, **characterised in that** the two radicals R are identical.

5. Polymers according to one or more of Claims 1 to 3, **characterised in that** one of the two radicals R is equal to H and the other is an aromatic or heteroaromatic ring system having 5 to 15 aromatic ring atoms.

6. Polymers according to one or more of Claims 1 to 5, **characterised in that** the polymer contains further structural elements selected from the group of the fluorene derivatives, the spirobifluorene derivatives, the dihydrophenanthrene derivatives, the cis- or trans-indenofluorene derivatives, the 1,4-phenylene derivatives, the 4,4'-biphenylylene derivatives, the 4,4"-terphenylylene derivatives, the 2,7- or 3,6-phenanthrene derivatives, the dihydropyrene or tetrahydropyrene derivatives, which may be substituted by R¹ or unsubstituted.

7. Polymers according to one or more of Claims 1 to 6, **characterised in that** the polymer contains further structural elements selected from the group of the R¹-substituted or unsubstituted condensed aromatic structures having 6 to 40 C atoms or tolan, stilbene or bisstyrylarylene derivatives.

8. Polymers according to one or more of Claims 1 to 7, **characterised in that** the polymer contains further structural elements selected from the group of the R¹-substituted or unsubstituted triarylamines, benzidines, N,N,N',N'-tetraaryl-paraphenylenediamines, triarylphosphines, phenothiazines, phenoxazines, dihydrophenazines, thianthrenes, dibenzo-p-dioxins, phenoxathiynes, carbazoles, azulenes, thiophenes, pyrroles, furans and further O-, S- or N-containing heterocycles having a high-lying HOMO.

9. Polymers according to one or more of Claims 1 to 8, **characterised in that** the polymer contains further structural elements selected from the group of the R¹-substituted or unsubstituted pyridines, pyrimidines, pyridazines, pyrazines, triazines, oxadiazoles, quinolines, quinoxalines or phenazines, the triarylboranes and further O-, S- or N-containing heterocycles having a low-lying LUMO.

10. Polymers according to one or more of Claims 1 to 9, **characterised in that** the polymer contains structural elements according to Claim 8 and according to Claim 9 which are bonded directly to one another.

11. Polymers according to one or more of Claims 1 to 10, **characterised in that** further structural elements are present which emit light from the triplet state with high efficiency at room temperature and exhibit electrophosphorescence instead of electrofluorescence and which contain at least one heavy atom having an atomic number of greater than 36.

12. Polymers according to Claim 11, **characterised in that**, in addition to the triplet emitter, structural elements selected from the group of the carbazoles, the bridged carbazole dimers, the ketones, phosphine oxides, sulfoxides, sulfones or the silanes are present.

13. Polymers according to one or more of Claims 1 to 12, **characterised in that** the proportion of units of the formula (1) is at least 5 mol%.

14. Polymer according to one or more of Claims 1 to 13, **characterised in that** the unit of the formula (1) is employed as polymer backbone or in combination with a polymer backbone and **in that**:
p is on each occurence equal to 0.

15. Polymer according to one or more of Claims 1 to 13, **characterised in that** the unit of the formula (1) is employed as hole-transporting unit and **in that**:
p is on each occurence, identically or differently, 0 or 1, where at least one p = 1;
o is on each occurence, identically or differently, 0, 1 or 2, where o is not equal to 0 if the corresponding p = 1;
X is on each occurence N-Ar.

16. Polymer according to one or more of Claims 1 to 13, **characterised in that** the unit of the formula (1) is employed as emitter and **in that**:
p is on each occurence, identically or differently, 0 or 1, where at least one p = 1;
o is on each occurence, identically or differently, 0, 1 or 2, where o is not equal to 0 if the corresponding p = 1;
X is on each occurence, identically or differently, -CR¹=CR¹-, -C≡C- or N-Ar, where at least one X is -CR¹=CR¹- or -C≡C-.

17. Use of bifunctional monomeric compounds of the formula (2), 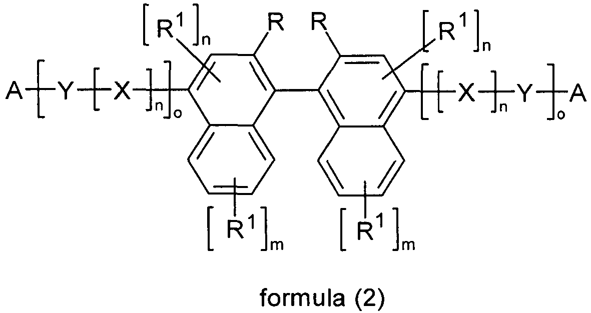 **characterised in that** the two functional groups A, identically or differently on each occurence, copolymerise under conditions of C-C or C-N linking and are selected from Cl, Br, I, O-tosylate, O-triflate, O-SO₂R², B(OR²)₂ and Sn(R²)₃, where R² has the same meaning as described in Claim 1, and where, in addition, two or more radicals R² may form a ring system with one another; the further symbols and indices have the same meaning as described in Claim 1, for the preparation of polymers.

18. Bifunctional monomeric compounds of the formula (2) according to Claim 17, with the proviso that the following compounds are excluded from the invention:

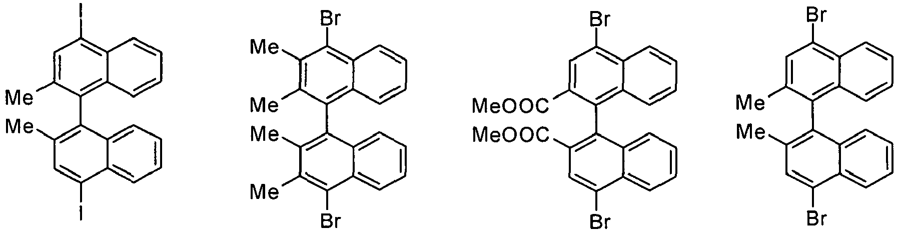

19. Mixtures (blends) of one or more polymers according to one or more of Claims 1 to 16 with further polymeric, oligomeric, dendritic or low-molecular-weight substances.

20. Mixtures according to Claim 19, **characterised in that** a compound which is able to emit light from the triplet state at room temperature is admixed.

21. Solutions and formulations composed of one or more polymers or blends according to one or more of Claims 1 to 16 and/or 19 to 20 in one or more solvents.

22. Use of a polymer or blend or solution according to one or more of Claims 1 to 16 and/or 19 to 21 in an organic electronic device.

23. Organic electronic device having one or more active layers, where at least one of these active layers comprises one or more polymers or blends according to one or more of Claims 1 to 16 and/or 19 to 20.

24. Organic electronic device according to Claim 23, selected from the group of the polymeric light-emitting diodes (PLEDs), organic integrated circuits (O-ICs), organic field-effect transistors (O-FETs), organic thin-film transistors (O-TFTs), organic solar cells (O-SCs), organic field-quench devices (O-FQDs), organic light-emitting transistors (O-LETs) or organic laser diodes (O-lasers).

## Revendications

1. Polymères contenant au moins 1% (pourcentage molaire) d'une première unité récurrente de la formule (1) : 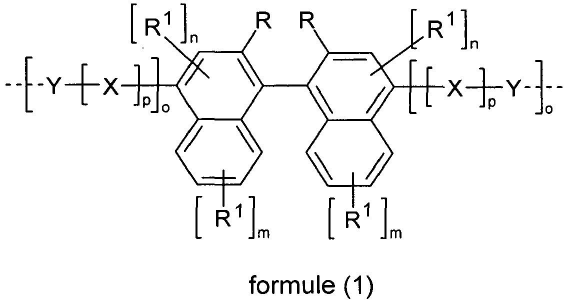 dans laquelle les symboles et indices utilisés présentent la signification qui suit :
R est pour chaque occurrence, de manière identique ou différente, H, une chaîne alkyle en chaîne droite comportant 1 à 40 atome(s) de C ou une chaîne alkyle ramifiée ou cyclique comportant 3 à 40 atomes de C, dont chacune peut être substituée par R¹ et où, en outre, un ou plusieurs atome(s) de C non adjacents peut/peuvent être remplacé(s) par N-R¹, O, S, O-CO-O, CO-O, -CR¹=CR¹- ou -C≡C-, étant entendu que des hétéroatomes ne sont pas liés directement à l'unité naphtyle, et où, en outre, un ou plusieurs atome(s) de H peut/peuvent être remplacé(s) par F, CI, Br, I ou CN, ou un système de cycle aromatique ou hétéroaromatique comportant 5 à 40 atomes de cycle aromatique, lequel peut également être substitué par un radical ou plusieurs radicaux R¹ ; les deux radicaux R peuvent ici également former un autre système de cycle l'un avec l'autre ; étant entendu qu'au moins l'un des deux radicaux R n'est pas égal à H ;
X est pour chaque occurrence, de manière identique ou différente, -CR¹=CR¹-, -C≡C- ou N-Ar ;
Y est pour chaque occurrence, de manière identique ou différente, un système de cycle aromatique ou hétéroaromatique divalent comportant 5 à 40 atomes de cycle aromatique, lequel peut être substitué par un radical ou plusieurs radicaux R¹ ou peut être non substitué ;
R¹ est pour chaque occurrence, de manière identique ou différente, H, F, Cl, Br, I, CN, N(R²)₂, Si(R²)₃, B(R²)₂, une chaîne alkyle ou alcoxy en chaîne droite comportant 1 à 40 atome(s) de C ou une chaîne alkyle ou alcoxy ramifiée ou cyclique comportant 3 à 40 atomes de C, où, en outre, un ou plusieurs atome(s) de C non adjacents peut/peuvent être remplacé(s) par N-R², O, S, O-CO-O, CO-O, -CR¹=CR¹- ou -C≡C- et où, en outre, un ou plusieurs atome(s) de H peut/peuvent être remplacé(s) par F, CI, Br, I ou CN, ou un groupe aryle, aryloxy ou hétéroaryle comportant 5 à 40 atomes de cycle aromatique, lequel peut également être substitué par un radical ou plusieurs radicaux non aromatiques R¹ ; deux ou plus des radicaux R¹ peuvent ici également former une système de cycle l'un avec l'autre ou les uns avec les autres ;
R² est pour chaque occurrence, de manière identique ou différente, H ou un radical hydrocarbone aliphatique ou aromatique comportant 1 à 20 atome(s) de C ;
Ar est pour chaque occurrence, de manière identique ou différente, un système de cycle aromatique ou hétéroaromatique monovalent comportant 5 à 40 atomes de cycle aromatique, lequel peut être substitué par R¹ ou peut être non substitué ;
n est pour chaque occurrence, de manière identique ou différente, 0 ou 1 ;
m est pour chaque occurrence, de manière identique ou différente, 0, 1, 2, 3 ou 4 ;
o est pour chaque occurrence, de manière identique ou différente, 0, 1 ou 2 ;
p est pour chaque occurrence, de manière identique ou différente, 0 ou 1 ;
le lien en pointillés représente ici la liaison dans le polymère ;
et de façon additionnelle, contenant au moins 1% (pourcentage molaire) d'une seconde unité récurrente, laquelle est soit identique à une unité récurrente de la formule (1), soit différente de cette même unité récurrente.

2. Polymères selon la revendication 1, **caractérisés en ce qu'**il s'agit de polymères conjugués ou partiellement conjugués.

3. Polymères selon la revendication 1 ou 2, **caractérisés en ce que**, pour les unités de la formule (1) :
R est pour chaque occurrence, de manière identique ou différente, H, une chaîne alkyle en chaîne droite comportant 1 à 10 atome(s) de C ou une chaîne alkyle ramifiée ou cyclique comportant 3 à 10 atomes de C, où, en outre, un ou plusieurs atome(s) de C non adjacents peut/peuvent être remplacé(s) par -CH=CH- ou -C≡C- et où, en outre, un ou plusieurs atome(s) de H peut/peuvent également être remplacé(s) par F ou CN, ou un système de cycle aromatique ou hétéroaromatique comportant 5 à 20 atomes de cycle aromatique, lequel peut également être substitué par un radical ou plusieurs radicaux non aromatiques R¹ ; les deux radicaux R peuvent ici également former un autre système de cycle l'un avec l'autre, étant entendu qu'au moins un radical R n'est pas égal à H ;
Y est pour chaque occurrence, de manière identique ou différente, un groupe aryle ou hétéroaryle divalent comportant 5 à 20 atomes de cycle aromatique, lequel peut être substitué par un radical ou plusieurs radicaux R¹ ;
Ar est pour chaque occurrence, de manière identique ou différente, un groupe aryle ou hétéroaryle monovalent comportant 5 à 20 atomes de cycle aromatique, lequel peut être substitué par R¹ ou non substitué ;
R¹ est pour chaque occurrence, de manière identique ou différente, H, F, N(R²)₂, une chaîne alkyle en chaîne droite comportant 1 à 10 atome(s) de C ou une chaîne alkyle ramifiée comportant 3 à 10 atomes de C, où, en outre, un ou plusieurs atome(s) de C non adjacents peut/peuvent être remplacé(s) par -CR¹=CR¹- ou -C≡C- et où, en outre, un ou plusieurs atome(s) de H peut/peuvent être remplacé(s) par F, ou un groupe aryle ou hétéroaryle comportant 5 à 20 atomes de cycle aromatique, lequel peut également être substitué par un radical ou plusieurs radicaux non aromatiques R¹ ; deux des radicaux R¹ ou plus peuvent ici également former un système de cycle l'un avec l'autre ou les uns avec les autres ;
n est pour chaque occurrence égal à 0 ;
m est pour chaque occurrence, de manière identique ou différente, 0, 1 ou 2 ;
o est pour chaque occurrence, de manière identique ou différente, 0 ou 1 ;
les autres symboles et indices sont comme défini selon la revendication 1.

4. Polymères selon une ou plusieurs des revendications 1 à 3, **caractérisés en ce que** les deux radicaux R sont identiques.

5. Polymères selon une ou plusieurs des revendications 1 à 3, **caractérisés en ce que** l'un des deux radicaux R est égal à H et l'autre est un système de cycle aromatique ou hétéroaromatique comportant 5 à 15 atomes de cycle aromatique.

6. Polymères selon une ou plusieurs des revendications 1 à 5, **caractérisés en ce que** le polymère contient d'autres éléments structurels choisis parmi le groupe constitué par les dérivés de fluorène, les dérivés de spirobifluorène, les dérivés de dihydrophénanthrène, les dérivés de cis- ou trans-indénofluorène, les dérivés de 1,4-phénylène, les dérivés de 4,4'-biphénylylène, les dérivés de 4,4"-ter-phénylylène, les dérivés de 2,7- ou 3,6-phénanthrène, les dérivés de dihydro-pyrène ou de tétrahydropyrène, lesquels peuvent être substitués par R¹ ou peuvent être non substitués.

7. Polymères selon une ou plusieurs des revendications 1 à 6, **caractérisés en ce que** le polymère contient d'autres éléments structurels choisis parmi le groupe constitué par les structures aromatiques condensées substituées par R¹ ou non substituées comportant 6 à 40 atomes de C ou les dérivés de tolane, de stilbène ou de bisstyrylarylène.

8. Polymères selon une ou plusieurs des revendications 1 à 7, **caractérisés en ce que** le polymère contient d'autres éléments structurels choisis parmi le groupe constitué par les triarylamines, les benzidines, les N,N,N',N'-tétraaryl-paraphénylènediamines, les triarylphosphines, les phénothiazines, les phénoxazines, les dihydrophénazines, les thianthrènes, les dibenzo-p-dioxines, les phénoxathiynes, les carbazoles, les azulènes, les thiophènes, les pyrroles, les furanes substitués par R¹ ou non substitués ainsi que d'autres hétérocycles contenant O, S ou N présentant une HOMO (orbite moléculaire occupée la plus haute) haute.

9. Polymères selon une ou plusieurs des revendications 1 à 8, **caractérisés en ce que** le polymère contient d'autres éléments structurels choisis parmi le groupe constitué par les pyridines, les pyrimidines, les pyridazines, les pyrazines, les triazines, les oxadiazoles, les quinolines, les quinoxalines ou les phénazines, les triarylboranes substitués par R1 ou non substitués ainsi que d'autres hétérocycles contenant O-, S- ou N présentant une LUMO (orbite moléculaire occupée la plus basse) basse.

10. Polymères selon une ou plusieurs des revendications 1 à 9, **caractérisés en ce que** le polymère contient d'autres éléments structurels selon la revendication 8 et selon la revendication 9, lesquels sont liés directement les uns aux autres.

11. Polymères selon une ou plusieurs des revendications 1 à 10, **caractérisés en ce que** d'autres éléments structurels sont présents, lesquels peuvent émettre de la lumière à partir de l'état triplet selon un rendement élevé à température ambiante et présentent une électrophosphorescence en lieu et place d'une électrofluorescence et lesquels contiennent au moins un atome lourd présentant un numéro atomique supérieur à 36.

12. Polymères selon la revendication 11, **caractérisés en ce que**, en plus d'un émetteur à l'état triplet, les éléments structurels choisis parmi le groupe constitué par les carbazoles, les dimères de carbazole pontés, les cétones, les oxydes de phosphine, les sulfoxydes, les sulfones ou les silanes sont présents.

13. Polymères selon une ou plusieurs des revendications 1 à 12, **caractérisés en ce que** la proportion d'unités de la formule (1) est d'au moins 5% (pourcentage molaire).

14. Polymère selon une ou plusieurs des revendications 1 à 13, **caractérisé en ce que** l'unité de la formule (1) est utilisée en tant que squelette de polymère ou en combinaison avec un squelette de polymère et **en ce que** :
p est pour chaque occurrence égal à 0.

15. Polymère selon une ou plusieurs des revendications 1 à 13, **caractérisé en ce que** l'unité de la formule (1) est utilisée en tant qu'unité de transport de trous et **en ce que** :
p est pour chaque occurrence, de manière identique ou différente, 0 ou 1, où au moins un p = 1 ;
o est pour chaque occurrence, de manière identique ou différente, 0, 1 ou 2, où o n'est pas égal à 0 si le p correspondant = 1 ;
X est pour chaque occurrence N-Ar.

16. Polymère selon une ou plusieurs des revendications 1 à 13, **caractérisé en ce que** l'unité de la formule (1) est utilisée en tant qu'émetteur et **en ce que** :
p est pour chaque occurrence, de manière identique ou différente, 0 ou 1, où au moins un p = 1 ;
o est pour chaque occurrence, de manière identique ou différente, 0, 1 ou 2, où o n'est pas égal à 0 si le p correspondant = 1 ;
X est pour chaque occurrence, de manière identique ou différente, -CR¹=CR¹-, -C≡C- ou N-Ar, où au moins un X est -CR¹=CR¹- ou -C≡C-.

17. Utilisation de composés monomériques bifonctionnels de la formule (2) : 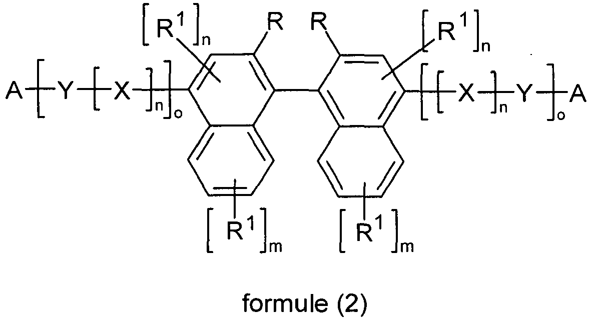 **caractérisée en ce que** les deux groupes fonctionnels A, de manière identique ou différente pour chaque occurrence, se copolymérisent sous des conditions de liaison C-C ou C-N et sont choisis parmi CI, Br, I, O-tosylate, O-triflate, O-SO₂R², B(OR²)₂ et Sn(R²)₃, où R² présente la même signification que décrit selon la revendication 1, et où, en outre, deux radicaux R² ou plus peuvent former un système de cycle l'un avec l'autre ou les uns avec les autres ; les autres symboles et indices présentent la même signification que décrit selon la revendication 1, pour la préparation de polymères.

18. Composés monomériques bifonctionnels de la formule (2) selon la revendication 17, étant entendu que les composés qui suivent sont exclus de l'invention :

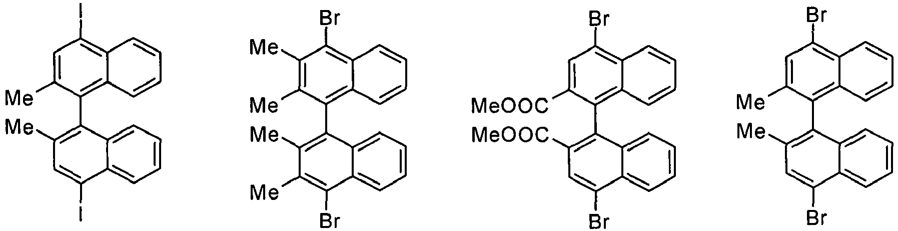

19. Mixtures (mélanges) d'un polymère ou de plusieurs polymères selon une ou plusieurs des revendications 1 à 16 avec d'autres substances polymériques, oligomériques, dendritiques ou de poids moléculaire faible.

20. Mixtures selon la revendication 19, **caractérisées en ce qu'**un composé qui dispose de la capacité d'émettre de la lumière à partir de l'état triplet à température ambiante est ajouté aux mixtures.

21. Solutions et formulations composées d'un ou de plusieurs polymère(s) ou mélange(s) selon une ou plusieurs des revendications 1 à 16 et/ou 19 à 20 dans un ou plusieurs solvant(s).

22. Utilisation d'un polymère ou d'un mélange ou d'une solution selon une ou plusieurs des revendications 1 à 16 et/ou 19 à 21 dans un dispositif électronique organique.

23. Dispositif électronique organique comportant une ou plusieurs couche(s) active(s), où au moins l'une de ces couches actives comprend un ou plusieurs polymère(s) ou mélange(s) selon une ou plusieurs des revendications 1 à 16 et/ou 19 à 20.

24. Dispositif électronique organique selon la revendication 23, choisi parmi le groupe constitué par les diodes émettrices de lumière polymériques (PLED), les circuits intégrés organiques (O-IC), les transistors à effet de champ organiques (O-FET), les transistors à film mince organiques (O-TFT), les cellules solaires organiques (O-SC), les dispositifs à extinction de champ organiques (O-FQD), les transistors à émission de lumière organiques (O-LET) ou les diodes laser organiques (O-laser).
